# EUROPÄISCHE PATENTANMELDUNG

(11) **EP 3 750 876 A1**
(43) Veröffentlichungstag der Anmeldung: **16.12.2020**
(21) Anmeldenummer: 19179843.8
(22) Anmeldetag: 13.06.2019
(51) Int. Cl.: C07D 211/12, C07D 211/74

(54) **VERFAHREN ZUR HERSTELLUNG VON TRIACETONAMIN, 2,2,4,6-TETRAMETHYLPIPERIDIN UND/ODER DEN SALZEN DES 2,2,4,6-TETRAMETHYLPIPERIDINS**

(71) Anmelder: Evonik Operations GmbH, 45128 Essen (DE)
(72) Erfinder: KERL, Thomas, 45130 Essen (DE); GÄRTNER, Felix, 45721 Haltern am See (DE); RIEB, Julia, 48143 Münster (DE); LUDWIG, Bettina, 45663 Recklinghausen (DE); MINKE, Katharina, 45128 Essen (DE)
(74) Vertreter: Evonik Patent Association

(57) **Zusammenfassung**

Die vorliegende Erfindung betrifft ein Verfahren zur Herstellung von Triacetonamin, 2,2,4,6-Tetramethylpiperidin und/oder den Salzen des 2,2,4,6-Tetramethylpiperidins. Das Verfahren zeichnet sich dadurch aus, dass die neben Triacetonamin im Rohprodukt enthaltenen Nebenprodukte besonders effizient isoliert und vor allem von dem ansonsten schwer abtrennbaren Nebenprodukt 2,2,4,6-Tetramethyl-2,3-dihydropyridin befreit werden. Das im Verfahren neben Triacetonamin erhaltene Nebenprodukt 2,2,4,6-Tetramethylpiperidin ist eine Base und ein Wertprodukt. Es kann als metallorganische, beispielsweise Grignard- oder Magnesium-LithiumVerbindung, zur Metallierung und Funktionalisierung von Aromaten eingesetzt werden.

## Beschreibung

Die vorliegende Erfindung betrifft ein Verfahren zur Herstellung von Triacetonamin, 2,2,4,6-Tetramethylpiperidin und/oder den Salzen des 2,2,4,6-Tetramethylpiperidins. Das Verfahren zeichnet sich dadurch aus, dass die neben Triacetonamin im Rohprodukt enthaltenen Nebenprodukte besonders effizient isoliert und vor allem von dem ansonsten schwer abtrennbaren Nebenprodukt 2,2,4,6-Tetramethyl-2,3-dihydropyridin befreit werden. Das im Verfahren neben Triacetonamin erhaltene Nebenprodukt 2,2,4,6-Tetramethylpiperidin ist eine Base und ein Wertprodukt. Es kann als metallorganische, beispielsweise Grignard- oder Magnesium-LithiumVerbindung, zur Metallierung und Funktionalisierung von Aromaten eingesetzt werden.

### Hintergrund der Erfindung

Triacetonamin (2,2,6,6-Tetramethyl-4-piperidinon; CAS-Nummer: 826-36-8; im Folgenden "TAA") ist ein wichtiges chemisches Intermediat, welches zur Synthese zahlreicher Folgeprodukte wie beispielsweise Lichtstabilisatoren (*hindered amine light stabilizers;* [HALS]), Oxidationsmitteln und Polymerisationsmoderatoren (z. B. Nitroxyl-Radikale) eingesetzt wird.

Die Herstellung von TAA aus Aceton und Ammoniak ist in Form verschiedener Verfahren dokumentiert. Dabei gliedern sich die Herstellungsverfahren in die direkte (einstufige) Synthese von TAA aus den Edukten, beispielsweise beschrieben in DE 24 29 937 A1, US 4,536,581 A, JPS54-88275 A oder in Zeitschrift für Naturforschung 1976, 328 - 337 und 338 - 345, sowie die indirekte (zweistufige) Synthese über Acetonin (2,2,4,4,6-Pentamethyl-1,2,5,6-tetrahydropyrimidin), beispielsweise beschrieben in DE 24 29 935 A1 oder DE 24 29 936 A1, oder über Phoron (2,6-Dimethyl-2,5-heptadien-4-on), z. B. beschrieben in DE 2 352 127 A1. Bei der zweistufigen TAA-Synthese über Acetonin wird dies zunächst ausgehend von Aceton und Ammoniak gebildet und reagiert dann in einem anschließenden Schritt unter Abspaltung von einem Äquivalent Ammoniak weiter zu TAA. Im Falle des Syntheseverfahrens über Acetonin werden jedoch immer beide Spezies (TAA und Acetonin) gleichzeitig gebildet, die Acetoninbildung ist allerdings kinetisch gegenüber der TAA-Bildung stark bevorzugt. In der "einstufigen" TAA-Synthese wird Acetonin lediglich nicht isoliert.

Die Herstellung von TAA ist grundsätzlich sowohl homogen katalysiert (meist durch Ammoniumsalze) als auch heterogen katalysiert (z. B. an sauren Ionentauschern) möglich.

Die meisten Schriften aus dem Stand der Technik beziehen sich auf homogen katalysierte Reaktionen. Am häufigsten werden dabei Calciumchlorid (z.B. in Chemical Industries 2003, 89, 559-564; Zeitschrift für Naturforschung 1976, 328 - 337 und 338 - 345), Ammoniumchlorid (z. B. in JP 2003-206277 A; JP 2001-31651 A; JPH4-154762 A) und Hydrazinderivate (z. B. in JPS54-88275 A, JPS54-112873 A) genannt. Bei Einsatz dieser Katalysatoren treten allerdings Probleme auf. So hat etwa der Einsatz von Calciumchlorid den Nachteil, dass eine sehr langsame Reaktion erfolgt. Im Falle des Ammoniumchlorids ist die Reaktionsgeschwindigkeit höher, das eingesetzte Chlorid korrodiert Stahl jedoch stark. Hydrazinderivate zeigen demgegenüber eine sehr hohe Toxizität.

Demgegenüber wurden auch Reaktionen an heterogenen Katalysatoren beschrieben, wie zum Beispiel in der DE 28 07 172 A1 und der CN 103224465 A.

Im Allgemeinen wird TAA in einer Matrix hergestellt, bei der das Aceton in großem Überschuss vorliegt und sowohl als Reaktionspartner als auch als Lösungsmittel dient. Daher ergibt sich am Ende der Reaktion ein Rohprodukt, welches neben TAA einen großen Anteil an Aceton, nicht umgesetzten Ammoniak, durch die Kondensation gebildetes Wasser und bei homogenkatalytischen Verfahren den Katalysator enthält. Darüber hinaus sind weitere Nebenkomponenten enthalten, wie z. B. acyclische Kondensationsprodukte (z. B. Diacetonalkohol, Diacetonamin, Mesityloxid, Phoron etc.), cyclische Kondensationsprodukte [z. B. Acetonin, 2,2,4,6-Tetramethyl-2,3-dihydropyridin (CAS-Nummer: 63681-01-6, im Folgenden "TMDH-Pyridin")] oder höhermolekulare Kondensationsprodukte ("Hochsieder").

Das Auftreten dieser Nebenprodukte stellt einen Nachteil der herkömmlichen Verfahren dar. Zwar können einige acyclische Additions- und Kondensationsprodukte (z. B. Diacetonalkohol [4-Hydroxy-4-methylpentan-2-on], Diacetonamin [4-Amino-4-methylpentan-2-on], Mesityloxid [4-Methylpent-3-en-2-on], Phoron etc.) anstatt Aceton als Edukte mit Ammoniak zu TAA umgesetzt werden. Dies kann man sich beispielsweise in Verfahren mit einem prozessinternen Recyclingstrom zunutze machen (DE 28 07 172 A1, CN 108383704 A, CN 108383776 A).

Ein Nachteil der herkömmlichen Verfahren zur TAA-Synthese besteht aber darin, dass die beschriebenen Nebenprodukte aufwendig aus dem Rohprodukt und vor allem von dem gewünschten Produkt TAA abzutrennen sind. Die Siedepunkte der typischen Nebenprodukte Diacetonalkohol (CAS-Nummer 123-42-2; Siedepunkt bei Normaldruck: 166 °C), Acetonin (CAS-Nummer 556-72-9; Siedepunkt ∼ 170 °C), Diacetonamin (CAS-Nummer 625-04-7, Siedepunkt ∼ 180 °C), Phoron (CAS-Nummer 504-20-1; Siedepunkt bei Normaldruck: 197 °C) liegen zwischen den Siedepunkten des Acetons (CAS-Nummer 67-64-1; Siedepunkt bei Normaldruck: 56 °C) bzw. Mesityloxids (CAS-Nummer 141-79-7; Siedepunkt bei Normaldruck: 130 °C) und des TAAs (Siedepunkt bei Normaldruck: 205 °C), und im Falle des Isophorons (CAS-Nummer 78-59-1; Siedepunkt bei Normaldruck: 215 °C) auch darüber.

Das problematischste Nebenprodukt bei der Synthese des TAA ist TMDH-Pyridin (Siedepunkt bei Normaldruck ∼ 170 °C). Es besitzt eine hohe Stabilität und kann deshalb im Gegensatz zu den meisten anderen Nebenprodukten der TAA-Synthese nicht wieder als Ausgangsstoff in der Synthese zum TAA eingesetzt werden. TMDH-Pyridin ist in diesem Sinne eine organosynthetische "Sackgasse". Aufgrund des TMDH-Pyridins gehen im TAA-Syntheseverfahren viele Acetonäquivalente verloren. Die kostenaufwändige Entsorgung des TMDH-Pyridins ist somit notwendig und erfolgt üblicherweise durch Verbrennung. Neben dem Entsorgungsaufwand führt sie demnach zu einem hohen CO₂-Ausstoß.

Darüber hinaus verkompliziert TMDH-Pyridin die destillative Reinigung des TAAs aus dem erhaltenen Reaktionsgemisch dadurch, dass es "verschmiert", das heißt einen breiten Siedebereich aufweist. Bei der destillativen Reinigung sind darum viele Destillationsstufen nötig, um unreagiertes Edukt wie Aceton oder andere Nebenprodukte wie z. B. Mesityloxid möglichst rein und frei von TMDH-Pyridin abzutrennen. Dies wiederum erfordert aufwendige Destillationsapparaturen, wie zum Beispiel eine Kolonne mit einer hohen Anzahl theoretischer Böden.

Daneben besteht eine Nachfrage nach organometallischen Reagenzien, die vielfältige Anwendung in der organischen Chemie finden, beispielsweise zur Metallierung und regioselektiven Funktionalisierung von Aromaten. Solche Reagenzien sind in der WO 2007/082911 A1 und der WO 2008/087057 A1 beschrieben. Zur Herstellung der in diesen Veröffentlichungen beschriebenen gemischten Magnesium/ Lithium-Amide und -Bisamide werden sterisch gehinderte Amine benötigt.

Es besteht deshalb der Bedarf nach einem effizienten Verfahren zur Synthese von TAA, welches die vorgenannten Probleme der herkömmlichen TAA-Syntheseverfahren nicht aufweist und insbesondere ein möglichst reines Produkt TAA liefert, wobei gleichzeitig die effiziente Verwertung der in der TAA-Synthese erhaltenen Nebenprodukte ermöglicht werden soll. Daneben besteht das Bedürfnis nach Aminen für die Herstellung metallorganischer Reagenzien.

### Abbildungen

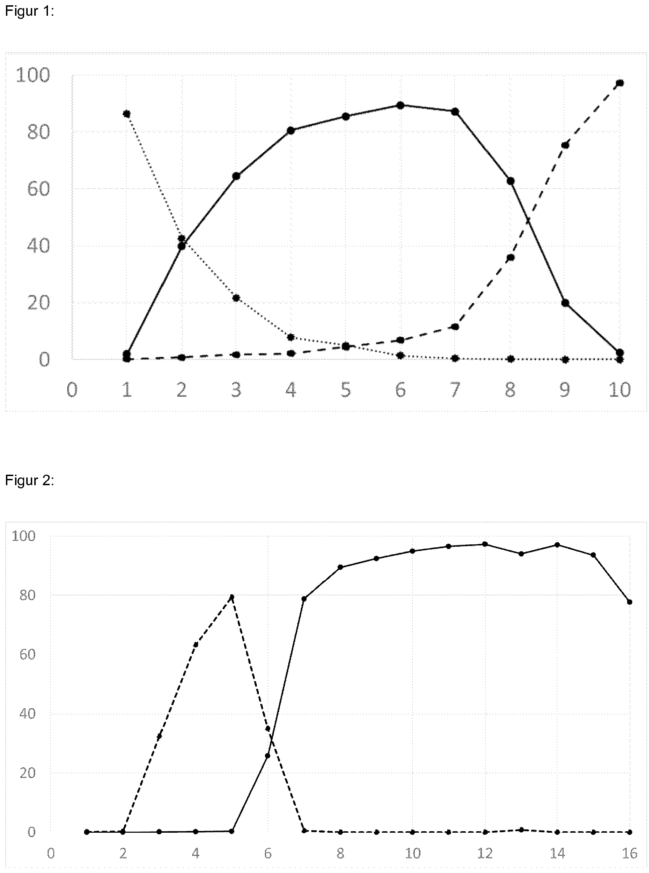

Abbildung 1 (= Figur 1) zeigt ausgewählte Komponenten des Gaschromatogramms des Kopfstromes 2. Die x-Achse bezeichnet die jeweilige Fraktion. Die y-Achse gibt den Anteil der jeweiligen ausgewählten Komponente in Flächenprozent des GC wieder: die durchgezogene Linie bezeichnet TMDH-Pyridin, die gestrichelte Linie bezeichnet Acetonin, die gepunktete Linie bezeichnet Mesityloxid.

Abbildung 2 (= Figur 2) zeigt ausfewählte Komponenten des Gaschromatogramms des Hydrierungsproduktes des Kopfstromes 1. Die x-Achse bezeichnet die jeweilige Fraktion. Die y-Achse gibt den Anteil der jeweiligen ausgewählten Komponente in Flächenprozent des GC wieder: die durchgezogene Linie bezeichnet *iso*-TEMP, die gestrichelte Linie bezeichnet 4-Methyl-pentan-2-on.

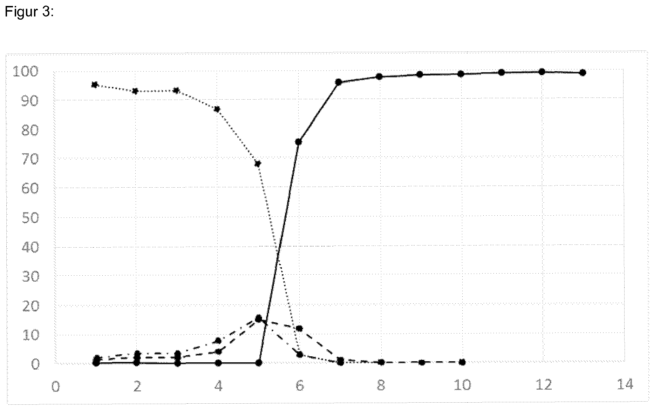

Abbildung 3 (= Figur 3) zeigt ausgewählte Komponenten des Gaschromatogramms des Hydrierungsproduktes des Kopfstromes 2. Die x-Achse bezeichnet die jeweilige Fraktion. Die y-Achse gibt den Anteil der jeweiligen ausgewählten Komponente in Flächenprozent des GC wieder: die durchgezogene Linie bezeichnet *iso*-TEMP, die gestrichelte Linie bezeichnet 4-Methyl-pentan-2-on, die gepunktete Linie bezeichnet Methanol, die abwechselnd gepunktete/gestrichelte Linie bezeichnet weitere Nebenkomponenten.

### Detaillierte Beschreibung der Erfindung

Die vorgenannte Aufgabe wird mittels der vorliegenden Erfindung gelöst. Es wurde nämlich überraschend festgestellt, dass ein besonders effizientes Verfahren zur Herstellung von TAA aus Aceton und Ammoniak dadurch ermöglicht wird, dass man aus dem erhaltenen Rohprodukt mindestens teilweise TMDH-Pyridin und Mesityloxid entfernt, TMDH-Pyridin in der so erhaltenen Mischung mindestens teilweise zu 2,2,4,6-Tetramethylpiperidin (im Folgenden "*iso*-TEMP") umsetzt und iso-TEMP von dem aus Mesityloxid entstehenden Hydrierungsprodukt 4-Methyl-pentan-2-on abtrennt. Das so erhaltene *iso*-TEMP kann danach zu den Salzen des *iso-*TEMPs, z. B. metallorganischen Verbindungen, umgesetzt werden.

Die vorliegende Erfindung betrifft demnach ein Verfahren zur Herstellung von Triacetonamin, 2,2,4,6-Tetramethylpiperidin und/oder einer metallorganischen Verbindung des 2,2,4,6-Tetramethylpiperidins, umfassend die folgenden Schritte
(a) Umsetzen von Ammoniak und Aceton in Gegenwart eines Katalysators **K₁** zu einem Rohprodukt **RP₁** umfassend Triacetonamin, TMDH-Pyridin und Mesityloxid,
(b) mindestens teilweise Abtrennung eines Rohprodukts **RP₁₁** umfassend TMDH-Pyridin und Mesityloxid aus **RP₁,** wobei **RP₁₁** eine gegenüber **RP₁** verringerte Menge an Triacetonamin aufweist,
   dadurch gekennzeichnet, dass
(c) **RP₁₁** mindestens teilweise, bevorzugt an einem heterogenen Katalysator **K₂** mit Wasserstoff, zu einem Rohprodukt **RP₁₂** umfassend 2,2,4,6-Tetramethylpiperidin und 4-Methyl-pentan-2-on umgesetzt wird,
(d) das in **RP₁₂** enthaltenen 2,2,4,6-Tetramethylpiperidin durch Destillation mindestens teilweise vom in **RP₁₂** enthaltenen 4-Methyl-pentan-2-on abgetrennt wird.

Es hat sich nämlich überraschenderweise gezeigt, dass die destillative Abtrennung des 4-Methyl-pentan-2-ons von *iso*-TEMP viel effizienter durchgeführt werden kann als die Abtrennung des Mesityloxids von TMDH-Pyridin. Dies erlaubt eine effizientere Isolierung der Wertprodukte. Daneben liefert das Verfahren neben dem Produkt Triacetonamin das Wertprodukt *iso*-TEMP, welches sich im Gegensatz zu TMDH-Pyridin vielfältig anwenden lässt, zum Beispiel als Ausgangsmaterial zur Herstellung metallorganischer Reagenzien.

### 1. Schritt (a)

Im Schritt (a) des erfindungsgemäßen Verfahrens werden Aceton und Ammoniak in Gegenwart eines Katalysators **K₁** umgesetzt.

Dabei wird ein Rohprodukt **RP₁** umfassend Triacetonamin erhalten. Dieses umfasst neben dem gewünschten Produkt TAA als Nebenprodukt Mesityloxid. Mesityloxid ist das einfachste Kondensationsprodukt, das stets zumindest in geringer Menge bei der Umsetzung von Ammoniak und Aceton zu TAA durch Aldolkondensation zweier Moleküle Aceton gebildet wird. Als höhermolekulares Kondensationsprodukte des Acetons mit sich und Ammoniak entsteht bei der Umsetzung im Schritt (a) auch TMDH-Pyridin. Dies ist deshalb vom Rohprodukt **RP₁** mit umfasst. Weitere Nebenprodukte, die gegebenenfalls von **RP₁** umfasst sind, sind ausgewählt aus der Gruppe bestehend aus Diacetonalkohol, Diacetonamin, Acetonin, Phoron, Isophoron, bevorzugt ausgewählt aus der Gruppe bestehend aus Diacetonalkohol, Diacetonamin, Acetonin, Isophoron.

Die Umsetzung in Schritt (a), wie auch das gesamte erfindungsgemäße Verfahren, kann kontinuierlich oder im Batch-Betrieb durchgeführt werden.

Im Falle des Batch-Betriebs werden bevorzugt alle Edukte zusammengegeben, dann wird die Reaktionsmischung erwärmt. Als Reaktor kommen dabei alle üblichen Reaktortypen in Frage, z. B. Rührreaktoren, Schlaufenreaktoren oder Reaktoren mit innenliegenden Wärmetauschern.

Im kontinuierlichen Betrieb werden bevorzugt alle Chemikalien gleichzeitig bei der Reaktionstemperatur zudosiert. Bei der kontinuierlichen Reaktion kann jeder dem Fachmann bekannte Reaktor eingesetzt werden, z. B. ein kontinuierliches Strömungsrohr, ein kontinuierlicher Rührkessel, eine Rührkesselkaskade, sowie mögliche Kombinationen aus diesen einzelnen Elementen. Dabei wird bevorzugt eine Kombination von einem oder mehreren Reaktoren mit internem oder externem Kreislauf, gefolgt von einem Nachreaktor mit Strömungsrohrcharakteristik, eingesetzt.

Die Reaktionszeit in Schritt (a) des erfindungsgemäßen Verfahrens im Batch-Betrieb liegt im Bereich von 1 bis 15 Stunden, bevorzugt im Bereich von 3 bis 9 Stunden, besonders bevorzugt im Bereich von 5 bis 7 Stunden. Die Reaktionszeiten für die kontinuierliche Verfahrensführung ergeben sich durch die Gesamt-Verweilzeit der Reaktanden im Reaktor und liegen in dem für den Batch-Betrieb genannten Bereich.

Die "*volume space velocity*" für Ammoniak im kontinuierlichen Betrieb liegt in Schritt (a) des erfindungsgemäßen Verfahrens insbesondere bei 0.01 bis 124.20 h⁻¹, bevorzugt 0.03 bis 5.25 h⁻¹, am bevorzugtesten bei 0.06 h⁻¹ (dies entspricht dem Volumen an Ammoniak, was pro Stunde und pro Volumen des Reaktors durch diesen Reaktor fließt, abgekürzt als "LHSV").

Die "*volume space velocity*" für Aceton im kontinuierlichen Betrieb liegt in Schritt (a) des erfindungsgemäßen Verfahrens insbesondere bei 0.15 bis 1.33 h⁻¹, bevorzugt 0.66 bis 1.17 h⁻¹ (dies entspricht dem Volumen an Aceton, was pro Stunde und pro Volumen des Reaktors durch diesen Reaktor fließt).

Die Umsetzung in Schritt (a) des erfindungsgemäßen Verfahrens kann in Gegenwart weiterer Lösungsmittel oder nur in Aceton, also ohne Zugabe weiterer Lösungsmittel, stattfinden. In den Fällen, in denen ein Lösungsmittel in Schritt (a) eingesetzt wird, können sämtliche Lösungsmittel eingesetzt werden, die die Reaktion nicht verhindern. Insbesondere sind die möglichen Lösungsmittel aliphatische Lösungsmittel, bevorzugt Pentan, Hexan, Heptan, Octan, Decan, Cyclohexan, Tetramethylsilan; aromatische Lösungsmittel, bevorzugt Benzol, Toluol, Xylol; Ether-Verbindungen, bevorzugt Diethylether, Dipropylether, Dibutylether, Methyl-*tert*-Butylether; halogenierte Lösungsmittel, bevorzugt Dichlormethan, Chloroform, Tetrachlormethan; Alkohole, bevorzugt Methanol, Ethanol, Propanol, Isopropanol, Butanol, *tert*-Butanol; Ester, bevorzugt Methylacetat, Ethylacetat, Propylacetat, Butylacetat. Besonders bevorzugt findet die Umsetzung in Schritt (a) des erfindungsgemäßen Verfahrens in Aceton, ohne Zugabe weiterer Lösungsmittel, statt.

Die Umsetzung in Schritt (a) wird vorzugsweise bei erhöhter Temperatur durchgeführt, insbesondere bei Temperaturen im Bereich von 20 °C bis 180°C, bevorzugt im Bereich von 40 °C bis 100 °C, bevorzugter im Bereich von 55 °C bis 90°C, noch bevorzugter im Bereich von 60 °C bis 90 °C, am bevorzugtesten bei 75 °C.

Die Umsetzung in Schritt (a) wird insbesondere entweder bei Eigendruck der Komponenten oder bei erhöhtem Druck durchgeführt. So wird die Umsetzung in Schritt (a) bevorzugt bei einem Druck im Bereich von 1 bis 16 bar, bevorzugter bei einem Druck im Bereich von 1 bis 15 bar, noch bevorzugter bei einem Druck im Bereich von 7 bis 14 bar, noch viel mehr bevorzugter bei einem Druck von 12 bar durchgeführt.

Ammoniak wird im Schritt (a) des erfindungsgemäßen Verfahrens bevorzugt als Reinstoff, d. h. als Gas zudosiert und liegt insbesondere während der Reaktion gelöst in Aceton bzw. gelöst im Reaktionsgemisch vor.

Aceton wird im Schritt (a) des erfindungsgemäßen Verfahrens bevorzugt als Reinstoff zudosiert. Alternativ kann Aceton verwendet werden, was in Schritt (b) einer vorher durchlaufenen Runde des erfindungsgemäßen Verfahrens abgetrennt wurde und auf diese Weise rezykliert wird. Es versteht sich von selbst, dass dann in der Reaktionslösung im Schritt (a) neben Aceton und Ammoniak Kondensationsprodukte des Acetons mit sich selbst oder Ammoniak vorliegen können. Diese können aus vorhergehenden Umsetzungsschritten von Ammoniak und Aceton zu TAA stammen.

Der Schritt (a) des erfindungsgemäßen Verfahrens wird in Gegenwart eines Katalysators **K₁** durchgeführt. Als Katalysator **K₁** sind alle im Stand der Technik für diesen Reaktionstyp genannten Katalysatoren einsetzbar. Der Katalysator **K₁** kann dabei homogen oder heterogen sein, ist bevorzugt jedoch heterogen.

Als homogener Katalysator **K₁** sind alle im Stand der Technik für diesen Reaktionstyp beschriebenen homogenen Katalysatoren geeignet, z. B. Brönstedt-Säuren, Salze dieser Säuren oder Lewissäuren, wie sie in der EP 2 706 056 A1 beschrieben sind.

Der Begriff "Brönsted-Säuren" im Sinne der Erfindung umfasst insbesondere Salzsäure, Schwefelsäure, Salpetersäure, organische Säuren (RCOOH) oder Sulfonsäuren (RSO₃H), wobei R ausgewählt ist aus der Gruppe bestehend aus gesättigten, ungesättigten, verzweigten, unverzweigten, ringgeschlossenen, offenkettigen aliphatischen, aromatischen, substituierten, unsubstituierten Kohlenwasserstoffresten. Substituierte Kohlenwasserstoffreste im Sinne der Erfindung sind Kohlenwasserstoffreste, die mit Heteroatomen substituiert sind, insbesondere Kohlenwasserstoffreste, welche mit einem oder mehreren Substituenten ausgewählt aus -OH, -NH, -CN, Alkoxy-, Halogenresten substituiert sind, bevorzugt mit einem oder mehreren Halogenresten substituiert sind, besonders bevorzugt mit einem oder mehreren Resten ausgewählt aus F, Cl, Br und I substituiert sind, ganz besonders bevorzugt mit einem oder mehreren Resten ausgewählt aus F und Cl substituiert sind.

"Salze einer Brönstedtsäure" im Sinne der Erfindung sind insbesondere Ammoniumsalze (d. h. Salze mit Ammoniak, Aminen, Hydrazinen, Hydroxylaminen) oder Phosphoniumsalze (d. h. Salze mit Phosphanen). Lewis-Säuren im Sinne der Erfindung sind insbesondere Verbindungen der 4. und der 13. Gruppe des Periodensystems, bevorzugt Halogenide (AlCl₃, BF₃, TiCl₄), Alkoxide [Al(OR**)₃, B(OR**)₃, Ti(OR**)₄] oder Alkylverbindungen (z. B. AlR**₃), wobei R** ausgewählt ist aus der Gruppe bestehend aus gesättigten, ungesättigten, verzweigten, unverzweigten, ringgeschlossenen, offenkettigen aliphatischen, aromatischen, substituierten, unsubstituierten Kohlenwasserstoffresten.

Lewis-Säuren im Sinne der Erfindung sind auch Salze Lewis-saurer Alkali- bzw. Erdalkalimetalle (z.B. CaCl₂, MgCl₂, LiCl).

Vorzugsweise ist in den Fällen, in denen **K₁** ein homogene Katalysator ist, dieser ausgewählt aus der Gruppe der Ammoniumsalze, insbesondere aus der Gruppe umfassend Salze aus Ammoniak und starken Brönsted-Säuren [z. B. Salzsäure, Schwefelsäure, Salpetersäure, organische Säuren (R***COOH) oder Sulfonsäuren (R***SO₃H), wobei R*** ausgewählt ist aus der Gruppe bestehend aus gesättigten, ungesättigten, verzweigten, unverzweigten, ringgeschlossenen, offenkettigen aliphatischen, aromatischen, substituierten, unsubstituierten Kohlenwasserstoffresten].

Bevorzugt ist in den Fällen, in denen **K₁** ein homogener Katalysator ist, dieser dann Ammoniumnitrat. Ammoniumnitrat hat den Vorteil, dass es günstig, nicht toxisch, halogenidfrei und damit weniger korrosiv ist.

Bevorzugt wird als Katalysator **K₁** jedoch ein heterogener Katalysator, insbesondere ein fester, saurer Katalysator eingesetzt, wie er beispielsweise in der DE 28 07 172 A1, der CN 103224465 A oder der DE 10 2010 062 804 A1 beschrieben ist. Dies sind solche Katalysatoren, die in dem Reaktionsmedium praktisch unlöslich sind. Bevorzugt wird dazu ein Katalysator genutzt, der anorganisch oder organisch ist und aktive saure Gruppen, bevorzugt Sulfonsäureestergruppen oder Phosphorsäureestergruppen, aufweist.

**K₁** ist demnach insbesondere ausgewählt aus der Gruppe bestehend aus Aluminiumhydrosilikaten vom Betonit- und/oder Montmorillonit-Typ, anorganischen Ionenaustauschern auf Aluminiumsilikat-Basis vom Zeolithtyp, Mordenittyp, Erionittyp oder auch mit Phosphorsäure bei 700 bis 1100 °C, wie in CA 772 201 beschrieben, behandelter Diatomerde.

Besonders für **K₁** bevorzugte heterogene Katalysatoren sind lonenaustauscherharze, insbesondere Kationenaustauscherharze. Diese sind bevorzugt sauer.

Als lonenaustauscherharze für **K₁** kommen insbesondere solche auf anorganischer (beispielsweise Siliciumdioxid) oder organischer (beispielsweise Polystyrol oder Polystyrolcopolymere, wie Styrol-Divinylbenzol-Copolymere), bevorzugt organischer, Basis, die protische Säuregruppen, insbesondere Alkylsulfonsäureestergruppen, Sulfonsäureestergruppen (-SO₃⁻), Phosphorsäureestergruppen, insbesondere Sulfonsäureestergruppen aufweisen, in Frage.

Ein besonders bevorzugter heterogener Katalysator für **K₁** ist ausgewählt aus der folgenden Gruppe:
- Ein Katalysator, der ein Polymerharz, insbesondere Polystyrol oder Styrol-Divinylbenzol-Copolymer, bevorzugt Polystyrol, und protische Säuren, insbesondere -SO₃⁻-Gruppen, als funktionelle Gruppen aufweist (käuflich erwerblich als "Amberlyst® 15", "Amberlite® 200", "Lewatit® SP 120" bzw. "Lewatit® K2621"); daneben ist auch Polystyrolsulfonat mit der CAS-Nummer: 28210-41-5 einsetzbar;
- Ein Katalysator, der protische Säuren, insbesondere Sulfonsäure in polymerer Form aufweist und perfluoriert sein kann (beschrieben in DE 10 2010 062 804 A1, US 4,831,146). Dieser kann zum Beispiel sulfoniertes Tetrafluorethylen (CAS-Nummer: 31175-20-9) oder eine feste geträgerte perfluorierte Sulfonsäure in polymerer Form mit Siliciumdioxid als Trägermaterial sein. Derartige Katalysatoren sind unter anderem unter den Handelsnamen Nafion®, Aciplex® F, Femion®, Neosepta®, Fumion® F erhältlich. Ein bevorzugter Katalysator ist Nafion® SAC-13. Bei Nafion® SAC-13 handelt es sich um poröse Siliciumdioxidpartikel, auf dem Nafion® in einer Beladung von etwa 13 Gew.-% adsorbiert worden ist;
- Poly(2-acrylamido-2-methyl-1-propanesulfonsäure), vertrieben als PolyAMPS® von Lubrizol.

Am bevorzugtesten wird als **K₁** ein heterogener Katalysator eingesetzt, der ein Polymerharz, insbesondere Polystyrol oder Styrol-Divinylbenzol-Copolymer, bevorzugt Polystyrol, und protische Säuren, insbesondere -SO₃⁻-Gruppen, als funktionelle Gruppen aufweist (käuflich erwerblich als "Amberlyst® 15", "Amberlite® 200", "Lewatit® SP 120" bzw. "Lewatit® K2621").

Die Einsatzverhältnisse der Edukte im Schritt (a) des erfindungsgemäßen Verfahrens können in weiten Bereichen gewählt werden, insbesondere wird Aceton im Überschuss zu Ammoniak eingesetzt. Bevorzugt beträgt das Molverhältnis von in Schritt (a) eingesetztem Aceton zu in Schritt (a) eingesetztem Ammoniak 3 : 1 bis 20 : 1, wobei ein Verhältnis von 6 : 1 bis 10 : 1 bevorzugt ist und ein Verhältnis von 7 : 1 besonders bevorzugt ist.

Die eingesetzte Menge an Katalysator **K₁** ist nicht besonders beschränkt und kann vom Fachmann bestimmt werden. Typischerweise kann, wenn der Katalysator ein homogener aus der Gruppe der Brönstedt-Säuren, Salze dieser Säuren oder Lewissäuren, bevorzugt ein Ammoniumsalz, noch bevorzugter Ammoniumnitrat ist, dieser im Molverhältnis von Ammoniak zu Katalysator, vorzugsweise Ammoniumnitrat, im Bereich von 1 : 0.8 bis 1 : 0.02 eingesetzt werden. Ganz besonders bevorzugt liegt in diesen Fällen das Molverhältnis von Aceton : Ammoniak : Ammoniumnitrat im Bereich von 7 bis 8 : 0.9 bis 1.1 : 0.085 bis 0.098.

In der bevorzugten Ausführungsform, in welcher ein fester, saurer Ionenaustauscher für **K₁** eingesetzt wird, kann dieser als Festbett eingesetzt werden, beispielsweise in einer Menge von 10 bis 20 Vol.-% bezogen auf die Gesamtmenge des in Schritt (a) des erfindungsgemäßen Verfahrens eingesetzten Acetons und - falls ein solches eingesetzt wurde - des Mesityloxids.

Im Anschluss an Schritt (a) des erfindungsgemäßen Verfahrens wird dann ein Rohprodukt **RP₁** erhalten, welches neben dem gewünschten Produkt Triacetonamin als Nebenprodukt Mesityloxid und TMDH-Pyridin umfasst. Gegebenenfalls kann **RP₁** auch unreagiertes Aceton umfassen. Daneben umfasst **RP₁** gegebenenfalls auch Wasser.

Daneben kann **RP₁** auch mindestens ein weiteres Nebenprodukt ausgewählt aus der Gruppe bestehend aus Diacetonalkohol, Diacetonamin, Acetonin, Phoron, Isophoron, bevorzugt ausgewählt aus der Gruppe bestehend aus Diacetonalkohol, Diacetonamin, Acetonin, Phoron, Isophoron, umfassen.

Der Anteil des TAA, TMDH-Pyridins, Mesityloxids und der genannten Nebenprodukte in **RP₁** ist nicht weiter beschränkt und ergibt sich aus der Stöchiometrie und den speziellen Reaktionsbedingungen. Der Anteil der jeweiligen Verbindung kann per GC bestimmt werden. Beispielsweise liegt nach der Reaktion ein Gemisch vor, in dem der Gehalt an Aceton bei 55 bis 60 Gew.-%, an Mesityloxid bei 10 Gew.-%, an TMDH-Pyridin bei 5 bis 6 Gew.-%, an der Summe aus Diacetonamin, Diacetonalkohol und Phoron bei 4 bis 6 Gew.-%, an TAA bei 14 bis 16 Gew.-%, und an höher als TAA siedenden Komponenten (zum Beispiel *iso*-Phoron) bei 3 bis 4 Gew.-% liegt, und der Anteil von Wasser 7 Gew.-% ist.

### 2. Schritt (b)

Im Schritt (b) des erfindungsgemäßen Verfahrens werden mindestens teilweise TMDH-Pyridin und Mesityloxid als Rohprodukt **RP₁₁** aus **RP₁** abgetrennt. Es wird also ein Rohprodukt **RP₁₁** erhalten, welches eine gegenüber **RP₁** verringerte Menge an Triacetonamin aufweist. Es ist dabei sinnvoll, in Schritt (b) Triacetonamin so gut wie möglich von TMDH-Pyridin zu trennen, da Triacetonamin als Wertstoff erhalten und nicht wie TMDH-Pyridin weiteren Reaktionen unterworfen werden soll.

Somit beträgt insbesondere der Gehalt an Triacetonamin in **RP₁₁** 10 Gew.-%, bevorzugt 1 Gew.-%, bevorzugter 0.1 Gew.-%, noch bevorzugter 0.1 Gew.-‰, noch mehr bevorzugter 0.01 Gew.-‰, noch viel mehr bevorzugter 1 ppm bezogen auf den Gehalt an Triacetonamin in **RP₁.**

Dabei kann der Schritt (b) des erfindungsgemäßen Verfahrens in jeder dem Fachmann bekannten Form erfolgen, durch die am Ende des Schrittes (b) ein abgetrenntes Rohprodukt **RP₁₁** umfassend TMDH-Pyridin und Mesityloxid erhalten wird. Bevorzugt erfolgt die mindestens teilweise Abtrennung von TMDH-Pyridin und Mesityloxid aus **RP₁** in Schritt (b) destillativ, noch bevorzugter in einer Destillationskolonne. In dieser bevorzugten Ausführungsform handelt es sich bei dem Rohprodukt **RP₁₁** um eine Fraktion, was bedeutet, dass **RP₁₁** als Destillat in mindestens einem destillativen Reinigungsschritt aus **RP₁** erhalten wurde.

In dieser Ausführungsform des erfindungsgemäßen Verfahrens liegt der bei der Destillation in Schritt (b) eingesetzte Temperaturbereich noch bevorzugter unterhalb der Siedetemperatur von Triacetonamin.

In Schritt (b) kann TAA dann ebenso nach dem Fachmann bekannten Verfahren aus **RP₁** entfernt werden. In der bevorzugten Ausführungsform des erfindungsgemäßen Verfahrens, in der Schritt (b) destillativ durchgeführt wird und bei der Destillation in Schritt (b) eingesetzte Temperaturbereich unterhalb der Siedetemperatur von Triacetonamin liegt, ist die Abtrennung des TAA noch einfacher von TMDH-Pyridin zu erreichen, da TAA im Sumpf der Destillation verbleibt und TMDH-Pyridin zusammen mit Mesityloxid als Destillat **RP₁₁** abgetrennt wird.

Es versteht sich von selbst, dass sich das im Schritt (b) des erfindungsgemäßen Verfahrens von **RP₁** abgetrennte Mesityloxid nicht zwangsläufig vollständig in **RP₁₁** wiederfinden muss. Natürlich kann aus **RP₁** auch in einem ersten Schritt eine gewisse Menge, insbesondere 50 Gew.-%, bevorzugt 80 Gew.-% bevorzugter 90 Gew.-% noch bevorzugter 99 Gew.-% des von **RP₁** umfassten Mesityloxids entfernt werden, bevorzugt ohne TMDH-Pyridin mitzuentfernen. Dadurch wird zunächst ein Rohprodukt **RP₁₁₁** erhalten, in dem der Gesamtgehalt an Mesityloxid gegenüber dem Gesamtgehalt an Mesityloxid in **RP₁** verringert ist. In einem zweiten Schritt wird dann aus **RP₁₁₁** TMDH-Pyridin zusammen mit Mesityloxid als **RP₁₁₂** entfernt.

In einer bevorzugten Variante des erfindungsgemäßen Verfahrens werden demnach aus dem Rohprodukt **RP₁** zunächst destillativ, bevorzugt in einer ersten Destillationskolonne **Kol₁** (für Leichtsieder), teilweise Mesityloxid abgetrennt und so ein Rohprodukt **RP₁₁₁** erhalten, so dass der Gesamtgehalt an Mesityloxid in **RP₁₁₁** (also dem Destillationsrückstand) gegenüber dem Gesamtgehalt an Mesityloxid in **RP₁** verringert ist und insbesondere ≤ 50 Gew.-%, bevorzugt ≤ 20 Gew.-% bevorzugter ≤ 10 Gew.-% noch bevorzugter ≤ 1 Gew.-% des Gesamtgehalts an Mesityloxid in **RP₁** entspricht. Es versteht sich von selbst, dass der Gehalt an Mesityloxid im so erhaltenen Destillationsrückstand **RP₁₁₁** zwar entsprechend dem vorher Gesagten verringert, aber > 0 ist.

Dann wird aus **RP₁₁₁** in einem zweiten destillativen Schritt, der bevorzugt in einer zweiten Destillationskolonne **Kol₂** (für Mittelsieder) erfolgt, TMDH-Pyridin zusammen mit Mesityloxid als Destillat **RP₁₁₂** abgetrennt. **RP₁₁₂** umfasst TMDH-Pyridin und Mesityloxid und wird in dieser bevorzugten Variante des erfindungsgemäßen Verfahrens dann als **RP₁₁** dem Schritt (c) des erfindungsgemäßen Verfahrens zugeführt.

In einer alternativen bevorzugten Variante des erfindungsgemäßen Verfahrens kann aus **RP₁₁₂** wieder destillativ, bevorzugt in einer dritten Destillationskolonne **Kol₃,** teilweise TMDH-Pyridin, welches immer noch Mesityloxid umfasst, als Destillat **RP₁₁₃** destilliert werden. **RP₁₁₃** umfasst TMDH-Pyridin und Mesityloxid und wird dann als **RP₁₁** dem Schritt (c) des erfindungsgemäßen Verfahrens zugeführt.

In weiteren alternativen bevorzugten Varianten des erfindungsgemäßen Verfahrens kann aus **RP₁₁₃** in einem vierten destillativen Schritt, bevorzugt einer vierten Destillationskolonne **Kol₄,** wiederum teilweise TMDH-Pyridin, welches immer noch einen geringen Anteil von Mesityloxid umfasst, destilliert werden. Dieses Destillat umfasst TMDH-Pyridin und Mesityloxid und kann dann, bevorzugt in einer oder mehreren weiteren Destillationskolonnen, immer wieder neuen Destillationsschritten unterworfen werden. Das in im letzten Destillationsschritt erhaltene Destillat wird dann als **RP₁₁** dem Schritt (c) des erfindungsgemäßen Verfahrens zugeführt.

Der Nutzen der vorliegenden Erfindung liegt darin begründet, dass eine effiziente Trennung von Mesityloxid von TMDH-Pyridin gerade mit Destillation nicht möglich ist. Somit werden auch in den bevorzugten Ausführungsformen der vorliegenden Erfindung, in der drei oder mehr destillative Schritte durchgeführt werden, stets Mesityloxid und TMDH-Pyridin als Mischung erhalten.

Das am Ende von Schritt (b) des erfindungsgemäßen Verfahrens erhaltene Reaktionsprodukt **RP₁₁,** bei dem es sich bevorzugt um eine Fraktion handelt, umfasst TMDH-Pyridin und Mesityloxid. Dabei ist der Gewichtsanteil von TMDH-Pyridin in **RP₁₁** insbesondere bei 50 bis 90 Gew.-%, bevorzugt 50 bis 85 Gew.-%, bevorzugter bei 53.75 bis 60 Gew.-%, und der von Mesityloxid > 0 bis 25 Gew.-%, bevorzugt 0.01 bis 25 Gew.-%, bevorzugter 0.1 bis < 10 Gew.-% wobei es sich bei den restlichen Verbindungen insbesondere um Aceton, Ammoniak, Diacetonalkohol, Diacetonamin, Acetonin, Phoron, Isophoron, bevorzugt Diacetonalkohol, Diacetonamin handelt.

Insbesondere liegt in **RP₁₁** das Verhältnis des Gesamtgewichts an TMDH-Pyridin zum Gesamtgewicht des Mesityloxid bei 25.6 : 1 bis 1 : 200, bevorzugt 16.5 : 1 bis 1 : 100, bevorzugt 8.5 : 1 bis 1 : 50, bevorzugter 1 : 1 bis 1 : 25.7, noch bevorzugter bevorzugt 1 : 5 bis 1 : 8.3.

Eine beispielhafte Zusammensetzung von **RP₁₁** ist 0.42 Gew.-% Aceton, 6.48 Gew.-% Mesityloxid, 3.11 Gew.-% Diacetonalkohol, 9.4 Gew.-% Diacetonamin, 53.75 Gew.-% TMDH-Pyridin, 23.35 Gew.-% Acetonin, 1.33 Gew.-% Phoron, 0.32 Gew.-% TAA mit 1.54 Gew.-% unbekannten Resten.

Eine alternative mögliche Zusammensetzung von **RP₁₁** (welche erhalten wird, wenn aus der vorgenannten Zusammensetzung nochmalig TMDH-Pyridin und Mesityloxid destilliert wird) ist 2.1 Gew.-% Aceton, 3.3 Gew.-% Mesityloxid, 84.8 Gew.-% TMDH-Pyridin, 7.5 Gew.-% Acetonin.

Gegebenenfalls findet während oder vor, bevorzugt vor, dem Schritt (b) auch eine Abtrennung des Katalysators **K₁** statt. Dies kann durch Zugabe einer Base geschehen. Zum Beispiel wird insbesondere NaOH zugegeben, wenn **K₁** ein Ammoniumsalz ist, und das dann ausgefallene Natriumnitrat wird anschließend abgetrennt.

Bei Verwendung eines heterogenen Katalysators erübrigt sich ein separater Reinigungsschritt oder ist zumindest deutlich einfacher, da beispielsweise bei Verwendung eines Festbettkatalysators dieser Katalysator im Reaktor verbleibt oder in anderen Fällen im Reaktionskessel verbleiben kann und/ oder durch Filtration abgetrennt werden kann. Auch deshalb ist die Verwendung eines heterogenen Katalysators für **K₁** vorzuziehen.

### 3. Schritt (c)

Im kennzeichnenden Schritt (c) des erfindungsgemäßen Verfahrens wird **RP₁₁,** also das von **RP₁₁** umfasste TMDH-Pyridin und Mesityloxid, mindestens teilweise, bevorzugt an einem heterogenen Katalysator **K₂** mit Wasserstoff, zu einer Rohprodukt **RP₁₂** umfassend 2,2,4,6-Tetramethylpiperidin und 4-Methyl-pentan-2-on umgesetzt.

Dem Fachmann sind Verfahren zur Umsetzung von TMDH-Pyridin zu 2,2,4,6-Tetramethylpiperidin bekannt. So beschreibt E. Matter in Helv. Chim. Acta 1948, 31, 612 - 622 entsprechende Verfahren unter Verwendung von Ameisensäure oder Natrium und Alkohol.

Bevorzugt findet die Umsetzung des TMDH-Pyridins zu 2,2,4,6-Tetramethylpiperidin an einem heterogenen Katalysator **K₂** mit Wasserstoff statt. Eine solche Umsetzung ist dem Fachmann ebenfalls bekannt und von N.C Hancox in Austr. J. Chem. 1953, 6, 143 - 151 beschrieben. Bevorzugt wird als heterogener Katalysator **K₂** ein Trägerkatalysator, wie er beispielsweise in der EP 3 048 096 A1 beschrieben ist, oder ein Vollkatalysator, wie beispielsweise in der EP 3 266 766 A1 beschrieben ist, eingesetzt.

Der Schritt (c) des erfindungsgemäßen Verfahrens kann ohne Zugabe weiterer Lösungsmittel oder unter Zugabe eines Lösungsmittels durchgeführt werden. In den Fällen, in denen ein Lösungsmittel in Schritt (c) eingesetzt wird, können sämtliche Lösungsmittel eingesetzt werden, die die Reaktion nicht verhindern. Insbesondere sind die möglichen Lösungsmittel aliphatische Lösungsmittel, bevorzugt Pentan, Hexan, Heptan, Octan, Decan, Cyclohexan, Tetramethylsilan; aromatische Lösungsmittel, bevorzugt Benzol, Toluol, Xylol; Ether-Verbindungen, bevorzugt Diethylether, Dipropylether, Dibutylether, Methyl-*tert*-Butylether; halogenierte Lösungsmittel, bevorzugt Dichlormethan, Chloroform, Tetrachlormethan; Alkohole, bevorzugt Methanol, Ethanol, Propanol, Isopropanol, Butanol, *tert*-Butanol; Ester, bevorzugt Methylacetat, Ethylacetat, Propylacetat, Butylacetat. Wird ein Lösungsmittel eingesetzt, ist Methanol vorzuziehen.

"Trägerkatalysator" bedeutet insbesondere, dass das Katalysatormetall von **K₂,** welches bevorzugt im elementaren Zustand vorliegt, auf einem dem Fachmann bekannten Träger aufgebracht ist, welcher insbesondere ausgewählt aus der Gruppe bestehend aus Aktivkohle, Calciumcarbonat, Aluminiumoxid, Titandioxid, insbesondere aus der Gruppe bestehend aus Aluminiumoxid, Aktivkohle sein kann. Das Katalysatormetall des Trägerkatalysators ist insbesondere ausgewählt aus der Gruppe bestehend aus V, Cr, Mo, Mn, Ni, Pd, Pt, Fe, Ru, Os, Co, Rh, Ir, Cu; bevorzugt ausgewählt aus der Gruppe bestehend aus V, Cr, Mo, Mn, Ni, Pd, Pt, Fe, Ru, Os, Co, Rh, Ir, Cu; bevorzugter ausgewählt aus der Gruppe bestehend aus Cr, Mo, Mn, Ni, Pd, Pt, Ru, Rh; noch bevorzugter ausgewählt aus der Gruppe bestehend aus Cr, Ni, Pd, Pt; am bevorzugtesten ausgewählt aus der Gruppe bestehend aus Ni, Pd.

Ein "Vollkatalysator" ist dem Fachmann bekannt und ist ein vollständig von dem katalytischen Material durchdrungener Formkörper. Er unterscheidet sich damit vom "Trägerkatalysator", bei dem die katalytisch aktive Komponente auf einem von der katalytisch aktiven Komponente verschiedenen Träger aufgebracht ist.

Das mindestens eine Metall im Vollkatalysator liegt insbesondere im elementaren Zustand oder als Verbindung des Metalls, beispielsweise als Oxid, Sulfid, vor, bevorzugt jedoch im elementaren Zustand.

Bevorzugt handelt es sich bei dem Vollkatalysator um einen Vollkatalysator aufweisend ein Metall ausgewählt aus der Gruppe bestehend aus Ag, V, Cr, Mo, Mn, Ni, Pd, Pt, Fe, Ru, Os, Co, Rh, Ir, Cu; insbesondere ausgewählt aus der Gruppe bestehend aus Ag, Fe, Cr, Mo, Mn, Ni, Co, Cu, Pd, Pt, Ru, Rh; bevorzugt ausgewählt aus der Gruppe bestehend aus Ag, Fe, Cr, Ni, Co, Cu, Pd, Pt; bevorzugter ausgewählt aus der Gruppe bestehend aus Ag, Fe, Ni, Co, Cu, Pd, Pt; noch bevorzugter ausgewählt aus der Gruppe bestehend aus Ag, Fe, Ni, Co, Cu; noch mehr bevorzugter ausgewählt aus der Gruppe bestehend Co, Ni; am bevorzugtesten Ni.

Der Vollkatalysator kann eine Legierung des Metalls sein (wobei das Metall u mindestens > 50 Gew.-% in der Legierung vorliegt, bezogen auf das Gesamtgewicht der Legierung) und beispielsweise außer dem Metall noch mindestens ein davon verschiedenes Metall bzw. Halbmetall ausgewählt aus Al, Si, Mg, Zn, Mo, Cr insbesondere Al, aufweisen.

Noch mehr bevorzugter ist der Vollkatalysator ausgewählt aus der Gruppe bestehend aus Raney-Kobalt, Raney-Kupfer, Raney-Silber, Raney-Eisen, Raney-Nickel, insbesondere ausgewählt aus Raney-Nickel, Raney-Kobalt, am bevorzugtesten ausgewählt aus Raney-Nickel.

Im Raney-Nickel ist der Anteil des Nickels bezogen auf das Gesamtgewicht des Raney-Nickels insbesondere mindestens > 50 Gew.-%, bevorzugt mindestens 60 Gew.-%, bevorzugter mindestens 70 Gew.-%, noch bevorzugter mindestens 80 Gew.-%, noch mehr bevorzugter mindestens 85 Gew.-%, noch viel mehr bevorzugter mindestens 90 Gew.-%, wobei das Raney-Nickel insbesondere daneben noch weitere von Nickel verschiedene Metalle und/oder Halbmetalle (wie beispielsweise Al, Mo, Si, Mg, Zn, Mo, Cr) in einer solchen Menge aufweist, dass die Summe der Gewichte aus Nickel und den übrigen Metallen und Halbmetallen 100 Gew.-% ergibt. Das Raney-Nickel kann insbesondere mit Zn, Mo, Cr, bevorzugt Mo dotiert sein, um die katalytischen Eigenschaften zu verbessern.

Im Raney-Kobalt ist der Anteil des Kobalts bezogen auf das Gesamtgewicht des Raney-Kobalts insbesondere mindestens > 50 Gew.-%, bevorzugt mindestens 60 Gew.-%, bevorzugter mindestens 70 Gew.-%, noch bevorzugter mindestens 80 Gew.-%, noch mehr bevorzugter mindestens 85 Gew.-%, noch viel mehr bevorzugter mindestens 90 Gew.-%, wobei das Raney-Kobalt insbesondere daneben noch weitere von Kobalt verschiedene Metalle und/oder Halbmetalle (wie beispielsweise Al, Mo, Si, Mg, Zn, Mo, Cr) in einer solchen Menge aufweist, dass die Summe der Gewichte aus Kobalt und den übrigen Metallen und Halbmetallen 100 Gew.-% ergibt. Das Raney-Kobalt kann insbesondere mit Zn, Mo, Cr, bevorzugt Mo dotiert sein, um die katalytischen Eigenschaften zu verbessern.

Im Raney-Kupfer ist der Anteil des Kupfers bezogen auf das Gesamtgewicht des Raney-Kupfers insbesondere mindestens > 50 Gew.-%, bevorzugt mindestens 60 Gew.-%, bevorzugter mindestens 70 Gew.-%, noch bevorzugter mindestens 80 Gew.-%, noch mehr bevorzugter mindestens 85 Gew.-%, noch viel mehr bevorzugter mindestens 90 Gew.-%, wobei das Raney-Kupfer insbesondere daneben noch weitere von Kupfer verschiedene Metalle und/oder Halbmetalle (wie beispielsweise Al, Mo, Si, Mg, Zn, Mo, Cr) in einer solchen Menge aufweist, dass die Summe der Gewichte aus Kupfer und den übrigen Metallen und Halbmetallen 100 Gew.-% ergibt. Das Raney-Kupfer kann insbesondere mit Zn, Mo, Cr, bevorzugt Mo dotiert sein, um die katalytischen Eigenschaften zu verbessern.

Im Raney-Silber ist der Anteil des Silbers bezogen auf das Gesamtgewicht des Raney-Silbers insbesondere mindestens > 50 Gew.-%, bevorzugt mindestens 60 Gew.-%, bevorzugter mindestens 70 Gew.-%, noch bevorzugter mindestens 80 Gew.-%, noch mehr bevorzugter mindestens 85 Gew.-%, noch viel mehr bevorzugter mindestens 90 Gew.-%, wobei das Raney-Silber insbesondere daneben noch weitere von Silber verschiedene Metalle und/oder Halbmetalle (wie beispielsweise Al, Mo, Si, Mg, Zn, Mo, Cr) in einer solchen Menge aufweist, dass die Summe der Gewichte aus Silber und den übrigen Metallen und Halbmetallen 100 Gew.-% ergibt. Das Raney-Silber kann insbesondere mit Zn, Mo, Cr, bevorzugt Mo dotiert sein, um die katalytischen Eigenschaften zu verbessern.

Im Raney-Eisen ist der Anteil des Eisens bezogen auf das Gesamtgewicht des Raney-Eisens insbesondere mindestens > 50 Gew.-%, bevorzugt mindestens 60 Gew.-%, bevorzugter mindestens 70 Gew.-%, noch bevorzugter mindestens 80 Gew.-%, noch mehr bevorzugter mindestens 85 Gew.-%, noch viel mehr bevorzugter mindestens 90 Gew.-%, wobei das Raney-Eisen insbesondere daneben noch weitere von Eisen verschiedene Metalle und/oder Halbmetalle (wie beispielsweise Al, Mo, Si, Mg, Zn, Mo, Cr) in einer solchen Menge aufweist, dass die Summe der Gewichte aus Eisen und den übrigen Metallen und Halbmetallen 100 Gew.-% ergibt. Das Raney-Eisen kann insbesondere mit Zn, Mo, Cr, bevorzugt Mo dotiert sein, um die katalytischen Eigenschaften zu verbessern.

Die Herstellung der Vollkatalysatoren ist dem Fachmann bekannt. Die Herstellung des Raney-Nickels ist zum Beispiel in den US 1,629,190, DE 20 2010 007837 U1 beschrieben. Dazu legiert man Ni mit Al, Si, Mg oder Zn (insbesondere mit Al, bevorzugt im Verhältnis 1:1) und löst aus der Legierung nach mechanischer Zerkleinerung mit Alkalien (insbesondere NaOH) das katalytisch unwirksame Metall (AI) mindestens teilweise heraus.

Entsprechend werden auch Raney-Kupfer, Raney-Kobalt, Raney Silber oder Raney-Eisen hergestellt (beschrieben zum Beispiel in der DE 20 2010 007837 U1).

In Schritt (c) des erfindungsgemäßen Verfahrens wird bevorzugt ein Trägerkatalysator als **K₂** eingesetzt, in dem bevorzugt das Katalysatormetall Pd ist und der Träger Aktivkohle aufweist.

Schritt (c) des erfindungsgemäßen Verfahrens wird insbesondere bei einer Temperatur von 20 °C bis 300 °C, bevorzugt 100 °C bis 200 °C; bevorzugter 140 °C durchgeführt.

Schritt (c) des erfindungsgemäßen Verfahrens wird insbesondere bei einem Druck von 1 bis 300 bar, bevorzugt 2 bis 100 bar, bevorzugter 3 bis 40 bar, noch bevorzugter 20 bis 25 bar durchgeführt.

Am Ende des Schrittes (c) des erfindungsgemäßen Verfahrens erhält man ein Rohprodukt **RP₁₂** umfassend neben *iso*-TEMP und 4-Methyl-pentan-2-on bevorzugt weitere Nebenprodukte wie Di-*iso*propylamin, *iso*-Propyl-*iso*-propylenamin, *N*-*iso*-Propyl-4-methylpentan-2-amin, *N*-*iso*-Propyl-4-methylpentan-2-imin, 2,2,4,5-Tetrahydro-2,2,4,6-tetramethyl-pyridin, Acetonin, *iso*-Propyl-(2,2,6,6-tetramethyl-piperidin-4-yl)amin.

*iso*-TEMP wird dabei sowohl als *cis-* als auch als *trans*-Isomer erhalten, welche jeweils als zwei Enantiomere vorliegen.

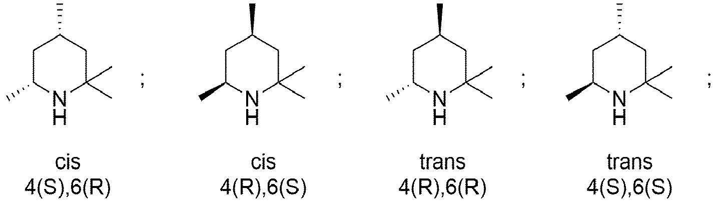

Dabei ist in vielen Fällen der Anteil der *cis*-Enantiomere höher als der der *trans*-Enantiomere, es tritt aber auch der Fall auf, dass der der Anteil der *cis*-Enantiomere niedriger als der der *trans-*Enantiomere ist.

Beispielsweise können Mischungen enthaltend alle vier Enantiomere des *iso*-TEMPs erhalten werden, in denen der Anteil der cis-Isomerer größer als der der trans-Isomere ist, und in denen der Anteil des 4(S),6(R) *cis*-Enantiomers den Anteil des 4(R),6(S) *cis*-Enantiomers überwiegt und der Anteil des 4(S),6(S) *trans*-Enantiomers den Anteil des 4(R),6(R) *trans*-Enantiomers überwiegt.

Ebenso können Mischungen enthaltend alle vier Enantiomere des *iso*-TEMPs erhalten werden, in denen der Anteil der cis-Isomerer kleiner als der der trans-Isomere ist, und in denen der Anteil des 4(S),6(R) *cis*-Enantiomers den Anteil des 4(R),6(S) *cis*-Enantiomers überwiegt und der Anteil des 4(S),6(S) *trans*-Enantiomers den Anteil des 4(R),6(R) *trans*-Enantiomers überwiegt.

Ebenso können Mischungen enthaltend alle vier Enantiomere des *iso*-TEMPs erhalten werden, in denen der Anteil der cis-Isomerer größer als der der trans-Isomere ist, und in denen der Anteil des 4(R),6(S) *cis*-Enantiomers den Anteil des 4(S),6(R) *cis*-Enantiomers überwiegt und der Anteil des 4(S),6(S) *trans*-Enantiomers den Anteil des 4(R),6(R) *trans*-Enantiomers überwiegt.

Ebenso können Mischungen enthaltend alle vier Enantiomere des *iso*-TEMPs erhalten werden, in denen der Anteil der cis-Isomerer kleiner als der der trans-Isomere ist, und in denen der Anteil des 4(R),6(S) *cis*-Enantiomers den Anteil des 4(S),6(R) *cis*-Enantiomers überwiegt und der Anteil des 4(S),6(S) *trans*-Enantiomers den Anteil des 4(R),6(R) *trans*-Enantiomers überwiegt.

Beispielsweise können Mischungen enthaltend alle vier Enantiomere des *iso*-TEMPs erhalten werden, in denen der Anteil der cis-Isomerer größer als der der trans-Isomere ist, und in denen der Anteil des 4(S),6(R) *cis*-Enantiomers den Anteil des 4(R),6(S) *cis*-Enantiomers überwiegt und der Anteil des 4(R),6(R) *trans*-Enantiomers den Anteil des 4(S),6(S) *trans*-Enantiomers überwiegt.

Ebenso können Mischungen enthaltend alle vier Enantiomere des *iso*-TEMPs erhalten werden, in denen der Anteil der cis-Isomerer kleiner als der der trans-Isomere ist, und in denen der Anteil des 4(S),6(R) *cis*-Enantiomers den Anteil des 4(R),6(S) *cis*-Enantiomers überwiegt und der Anteil des 4(R),6(R) *trans*-Enantiomers den Anteil des 4(S),6(S) *trans*-Enantiomers überwiegt.

### 4. Schritt (d)

Im kennzeichnenden Schritt (d) des erfindungsgemäßen Verfahrens wird das in **RP₁₂** enthaltenen 2,2,4,6-Tetramethylpiperidin vom in **RP₁₂** enthaltenen 4-Methyl-pentan-2-on mindestens teilweise abgetrennt.

Die Abtrennung von 2,2,4,6-Tetramethylpiperidin aus **RP₁₂** erfolgt mit Destillation, welche bevorzugt in einer nachfolgenden Destillationskolonne stattfindet, erfolgen.

Die Vorteile des erfindungsgemäßen Verfahrens verwirklichen sich, da der Schritt (d) des erfindungsgemäßen Verfahrens mit Destillation durchgeführt wird. Es hat sich nämlich gezeigt, dass eine viel bessere Abtrennung des 4-Methyl-pentan-2-on möglich ist, wenn diese aus einer Lösung umfassend 2,2,4,6-Tetramethylpiperidin anstatt TMDH-Pyridins stattfindet.

Der weitere überraschende Effekt des erfinderischen Verfahrens ist jedoch, dass man nach Abschluss des erfinderischen Verfahrens anstatt wie herkömmlich TMDH-Pyridin den Wertstoff *iso*-TEMP erhält. Dieser kann jedem dem Fachmann bekannten Verwendungszweck zugeführt werden. Bevorzugt wird *iso*-TEMP der im Folgenden diskutierten Verwendung [nämlich Schritt (e) des erfindungsgemäßen Verfahrens] zugeführt.

### 5. Verwendung des 2,2,4,6-Tetramethylpiperidins als Elektrophil

2,2,4,6-Tetramethylpiperidin ist ein Wertstoff und findet vielfältige Anwendung in der organischen Chemie.

Eine Anwendung solcher Amine ist der Einsatz als metallorganische Addukte. So werden zur Metallierung von Aromaten etwa Metallamide, beispielsweise Lithiumamide, Magnesiumamide oder Amidozinkate, aber auch gemischtmetallische organische Verbindungen eingesetzt [M. Schlosser, Angew. Chem. Int. Ed. 2005, 44, 376-393; A. Turck, N. Ple, F. Mongin, G. Queguiner, Tetrahedron 2001, 57, 4489-4505; F. Mongin, G. Queguiner, Tetrahedron 2001, 57, 4059-4090, M. Schlosser, Eur. J. Org. Chem. 2001, 21, 3975-3984; D. M. Hodgson, C. D. Bray, N. D. Kindon, Org. Lett. 2005, 7, 2305-2308; J-C. Plaquevent, T. Perrard, D. Cahard, Chem. Eur. J. 2002, 8, 3300-3307; C. -C. Chang, M. S. Ameerunisha, Coord. Chem. Rev. 1999, 189, 199-278; K. W. Henderson, W. J. Kerr, Chem.-A Eur. J. 2001, 7, 3430-3437; K. W. Henderson, W. J. Kerr, J. H. Moir, Tetrahedron 2002, 58, 4573-4587; M. C. Whisler, S. MacNeil, V. Snieckus, P. Beak, Angew. Chem. Int. Ed. 2004, 43, 2206-2225; G. Queguiner, F. Marsais, V. Snieckus, J. Epsztajn, Adv. in Het. Chem. 1991, 52, 187-304; M. Veith, S. Wieczorek, K. Fries, V. Huch, Z. Anorg. Allg. Chem. 2000, 626, 1237-1245].

Beispielsweise beschreiben WO 2008/087057 A1 und EP 1 810 974 A1 Lithiummagnesiumverbindungen, die von 2,2,6,6-Tetramethylpiperidin (im Folgenden "TEMP") ausgehen, nämlich TEMP₂Mg·2LiCl und TEMPMgCl·LiCl.

Es hat sich nun gezeigt, dass sich das im Schritt (d) erhaltene *iso*-TEMP als Ausgangsstoff für die Herstellung solcher (gemischt)metallorganischer Addukte eignet.

In einem bevorzugten Aspekt des erfindungsgemäßen Verfahrens wird deshalb in einem Schritt (e) das in Schritt (d) abgetrennte *iso*-TEMP mit einem Reagenz der Formel Me_{A}^{m1}Rₙ₁Y¹ₙ₂·zMe_{B}^{m2}Y²ₙ₃ in einem Lösungsmittel umgesetzt,
wobei m1, n1, m2, n3 unabhängig voneinander jeweils eine ganze Zahl > 0 sind, und wobei n2, z unabhängig voneinander jeweils eine ganze Zahl ≥ 0 sind,
wobei m1 die Wertigkeit des Metalls Me_{A} angibt und wobei m2 die Wertigkeit des Metalls Me_{B} angibt, wobei gilt, dass m1 + z·m2 = n1 + n2 + z·n3,
und bevorzugt m1 = 2 (dann ist das Metall Me_{A} zweiwertig), m2 = 1 (dann ist das Metall Me_{B} einwertig), n1 = n2 = n3 = 1, z = 0 oder z = 1, noch bevorzugter z = 1,
wobei R ein Kohlenwasserstoffrest ausgewählt aus Alkylgruppe, Alkenylgruppe, Arylgruppe, Alkinylgruppe ist und bevorzugt eine Alkylgruppe ist, noch bevorzugter eine Alkylgruppe mit 1 bis 6 Kohlenstoffatomen, bei der es sich am bevorzugtesten um Methyl oder *iso*-Propyl handelt, ist,
wobei Me_{A}, Me_{B} unabhängig voneinander jeweils ein Metall ausgewählt aus der Gruppe bestehend aus Li, Mg, Zn, Zr, Ti, Cu, Au, Ag, Hg, Cd, bevorzugt aus der Gruppe Li, Mg, Zn, Zr, Cu, besonders bevorzugt aus der Gruppe Li, Mg ist, am allerbevorzugtesten Me_{A} = Mg und Me_{B} = Li ist,
und wobei Y¹ und Y² unabhängig voneinander aus der Gruppe bestehend aus F, Cl, Br, I, CN, SCN, NCO, ClO₃, ClO₄, BrO₃, BrO₄, IO₃, IO₄₁ NO₃, BF₄, PF₆, Wasserstoff, ein Carboxylat der allgemeinen Formel R^{x}CO₂, ein Alkoholat der allgemeinen Formel OR^{x}; ein Thiolat der allgemeinen Formel SR^{x}, R^{x}P(O)O₂, or SCOR^{x}, SCSR^{x}, O₂SR^{x}, O₃SR^{x}, NO₂ ausgewählt sind,
wobei R^{x} ausgewählt ist aus der Gruppe bestehend aus unsubstituierter oder substituierter Arylest oder Heteroarylrest mit einem oder mehreren Heteroatomen, verzweigter oder unverzweigter Alkylrest, Cycloalkylrest, Alkenylrest, Alkinylrest, Wasserstoff oder als Addukt mit einem Lösungsmittel, bevorzugt Y¹ und Y² unabhängig voneinander aus der Gruppe bestehend aus Cl, Br, I ausgewählt sind, noch bevorzugter Y¹ = Y² = Cl,
wodurch eine metallorganische Verbindung des iso-TEMPs der allgemeinen Formel Me_{A}^{m1}[R_{iso-TEMP}]ₙ₁Y¹ₙ₂·zMe_{B} ^{m2}Y²ₙ₃ mit R_{iso-TEMP} = 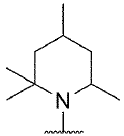 erhalten wird.

In einer bevorzugten Ausführungsform des erfindungsgemäßen Verfahrens wird deshalb in einem Schritt (e) das in Schritt (d) abgetrennte *iso*-TEMP mit einem Reagenz der Formel MgRY·LiY oder MgRY mit R = Alkylgruppe, bevorzugt mit 1 bis 6, bevorzugter 1 bis 4, noch bevorzugter 1 bis 3 Kohlenstoffatomen, bei der es sich noch bevorzugter um *iso*-Propyl oder Methyl handelt, und Y = Cl, Br, I, bevorzugt Y = Cl, Br, bevorzugter Y = Cl in einem Lösungsmittel umgesetzt.

In einer noch bevorzugteren Ausführungsform des erfindungsgemäßen Verfahrens wird in einem Schritt (e) das in Schritt (d) abgetrennte *iso*-TEMP mit einem Reagenz der Formel *iso*-PropylMgCl·LiCl oder MethylMgCl umgesetzt.

Das in Schritt (e) des erfindungsgemäßen Verfahrens verwendete Lösungsmittel ist nicht weiter beschränkt und bevorzugt aus der Gruppe bestehend aus cyclischen, unverzweigten, verzweigten Mono- oder Polyethern, Thioethern, Aminen, Phosphinen, bevorzugt Tetrahydrofuran (THF), 2-Methyltetrahydrofuran, Dibutylether, Diethylether, tert-Butylmethylether, Dimethoxyethan, Dioxane, bevorzugt 1,4-Dioxan, Triethylamin, Ethyldi*iso*propylamin, Dimethylsulfid, Dibutylsulfid; cyclische Amide, bevorzugt *N*-Methyl-2-pyrrolidon (NMP), *N*-Ethyl-2-pyrrolidon (NEP), *N*-Butyl-2-pyrrolidon (NBP); cyclische, unverzweigte oder verzweigte Haloalkane und Haloalkene, bevorzugt Dichlormethan, 1,2-Dichlorethan, CCl₄; Harnstoffe, bevorzugt *N*,*N'*-Dimethylpropylenharnstoff (DMPU); aromatische, heteroaromatische oder aliphatische Kohlenwasserstoffe, bevorzugt Benzol, Toluol, Xylol, Pyridin, Pentan, Cyclohexan, Hexan, Heptan; Hexamethylphosphorsäuretriamid (HMPA), CS₂.

Besonders bevorzugt ist das in Schritt (e) eingesetzte Lösungsmittel aus der Gruppe bestehend aus Tetrahydrofuran (THF), 2-Methyltetrahydrofuran, Dibutylether, Diethylether, *tert*-Butylmethylether, Dimethoxyethan, 1,4-Dioxan, Triethylamin, Ethyldi*iso*propylamin, Dimethylsulfid, Dibutylsulfid, *N*-Methyl-2-pyrrolidon (NMP), *N*-Ethyl-2-pyrrolidon (NEP), *N*-Butyl-2-pyrrolidon (NBP), Dichlormethan, 1,2-Dichlorethan, CCl₄, *N*,*N*'-Dimethylpropylenharnstoff (DMPU), Benzol, Toluol, Xylol, Pyridin, Pentan, Cyclohexan, Hexan, Heptan; Hexamethylphosphorsäuretriamid (HMPA), CS₂ ausgewählt, ganz besonders bevorzugt ist es THF oder Diethylether, noch bevorzugter THF.

Die Verfahrensbedingungen im Schritt (e) des erfindungsgemäßen Verfahrens sind dem Fachmann bekannt und in der Literatur beschrieben (WO 2008/087057 A1, EP 1 810 974 A1).

Für Magnesiumamide beispielsweise beschreiben dies M-X. Zhang, P. E. Eaton, Angew. Chem. Int. Ed. 2002, 41, 2169-2171; Y. Kondo, Y. Akihiro, T. Sakamoto, J. Chem. Soc, Perkin Trans. 1: Org. Bio-Org. Chem. 1996, 19, 2331-2332; P. E. Eaton, C. H. Lee, Y. Xiong, J. Am. Chem. Soc. 1989, 111, 8016-8018; P. E. Eaton, M-X. Zhang, N. Komiya, C-G. Yang, I. Steele, R. Gilardi, Synlett 2003, 9, 1275-1278.; P. E. Eaton, R. M. Martin, J. Org. Chem. 1988, 53, 2728-2732; M. Shilai, Y. Kondo, T. Sakamoto, J. Chem. Soc, Perkin Trans. 1: Org. Bio-Org. Chem. 2001, 4, 442; P. Knochel, W. Dohle, N. Gommermann, F. F. Kneisel, F. Kopp, T. Korn, I. Sapountzis, V. A. Vu, Angew. Chem. 2003, 115, 4438-4456.

Für Zinkate beschreiben dies Y. Kondo, M. Shilai, M. Uchiyama, T. Sakamoto, J. Am. Chem. Soc. 1999, 121, 3539-3540; T. Imahori, M. Uchiyama, T. Sakamoto, Y. Kondo, Chem. Comm. 2001, 23, 2450-2451.

Für auf Titan und Zirkonium basierende Verbindungen beschreiben dies B. Weidman und D. Seebach, Angew. Chem. 1983, 95, 12-26.

Für Cuprate beschreiben dies beispielsweise J.F. Normant, Synthesis 1972, 2, 63-80 und C.G. Chavdarian, C.H. Heathcock, J. Am. Chem. Soc. 1975, 97, 3822-3823.

Für Aurate beschreiben das beispielsweise P. Lange, A. Schier, J. Riede, H. Schmidbaur, Z. Naturforsch. 1994, 49b, 642-646 und L. Cao, M.C. Jennings, R.J. Puddephatt, Inorg. Chem. 2007, 46, 1361-1368.

Für organische Silberverbindungen beschreiben das beispielsweise X. Xu, W. Xu, J. Wu, J. He, H. Xu, Org. Biomol. Chem. 2016, 14, 9970-9973 und C. Wang, Q. Chen, Q. Guo, H. Liu, Z. Xu, Y. Liu, M. Wang, R. Wang, J. Org. Chem. 2016, 81, 5782-5788.

Für organische Quecksilberverbindungen beschreibt das H.O. Calvery, Org. Synth. 1929, 9, 54 und R.C. Larock, Angew. Chem. 1978, 90, 28-38.

Für organische Cadmiumverbindungen beschreiben das J.R. Sanders und E.C. Ashby, J. Organomet. Chem. 1971, 25, 277-286.

Der Schritt (e) des erfindungsgemäßen Verfahrens wird demnach bevorzugt bei einer Temperatur im Bereich von - 50 °C bis 25 °C, bevorzugt im Bereich von - 40 °C bis 0 °C durchgeführt. Typischerweise wird die Verbindung Me_{A}^{m1}Rₙ₁Y¹ₙ₂·zMe_{B}^{m2}Y²ₙ₃ in dem betreffenden Lösungsmittel vorgelegt und dann das in Schritt (e) erhaltene *iso*-TEMP hinzugegeben.

Die in Schritt (e) des erfindungsgemäßen Verfahrens erhaltenen metallorganischen Verbindungen des 2,2,4,6-Tetramethylpiperidins eignen sich zum Einsatz für vielfältige Reaktionen der organischen Chemie, insbesondere zur Verwendung in der Reaktion mit einem Elektrophil oder zur Deprotonierung von Substraten, die stabilisierte oder unstabilisierte Carbanionen bilden können.

In einem Aspekt der vorliegenden Erfindung betrifft diese demnach auch eine metallorganische Verbindung des iso-TEMPs der allgemeinen Formel Me_{A}^{m1}[R_{iso-TEMP}]ₙ₁Y¹ₙ₂·zMe_{B}^{m2}Y²ₙ₃ mit R_{iso-TEMP} = 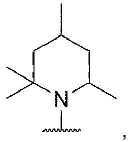 wobei m1, n1, m2, n3 unabhängig voneinander jeweils eine ganze Zahl > 0 sind, und wobei n2, z unabhängog voneinander jeweils eine ganze Zahl ≥ 0 sind,
wobei m1 die Wertigkeit des Metalls Me_{A} angibt und wobei m2 die Wertigkeit des Metalls Me_{B} angibt, wobei gilt, dass m1 + z·m2 = n1 + n2 + z·n3,
und bevorzugt m1 = 2 (dann ist das Metall Me_{A} zweiwertig), m2 = 1 (dann ist das Metall Me_{B} einwertig), n1 = n2 = n3 = 1, z = 0 oder z = 1, noch bevorzugter z = 1,
wobei Me_{A}, Me_{B} unabhängig voneinander jeweils ein Metall ausgewählt aus der Gruppe bestehend aus Li, Mg, Zn, Zr, Ti, Cu, Au, Ag, Hg, Cd, bevorzugt aus der Gruppe Li, Mg, Zn, Zr, Cu, besonders bevorzugt aus der Gruppe Li, Mg ist, am allerbevorzugtesten Me_{A} = Mg und Me_{B} = Li ist,
und wobei Y¹ und Y² unabhängig voneinander aus der Gruppe bestehend aus F, Cl, Br, I, CN, SCN, NCO, ClO₃, ClO₄, BrO₃, BrO₄, IO₃, IO₄₁ NO₃, BF₄, PF₆, Wasserstoff, ein Carboxylat der allgemeinen Formel R^{x}CO₂, ein Alkoholat der allgemeinen Formel OR^{x}; ein Thiolat der allgemeinen Formel SR^{x}, R^{x}P(O)O₂, or SCOR^{x}, SCSR^{x}, O₂SR^{x}, O₃SR^{x}, NO₂ ausgewählt sind,
wobei R^{x} ausgewählt ist aus der Gruppe bestehend aus unsubstituierter oder substituierter Arylest oder Heteroarylrest mit einem oder mehreren Heteroatomen, verzweigter oder unverzweigter Alkylrest, Cycloalkylrest, Alkenylrest, Alkinylrest, Wasserstoff oder als Addukt mit einem Lösungsmittel, bevorzugt Y¹ und Y² unabhängig voneinander aus der Gruppe bestehend aus Cl, Br, I ausgewählt sind, noch bevorzugter Y¹ = Y² = Cl.

Bevorzugt liegt die metallorganische Verbindung des iso-TEMPs der allgemeinen Formel Me_{A}^{m1}[R_{iso-TEMP}]ₙ₁Y¹ₙ₂·zMe_{B}^{m2}Y²ₙ₃ in einem Lösungsmittel vor, welches insbesondere aus der Gruppe bestehend aus cyclischen, unverzweigten, verzweigten Mono- oder Polyethern, Thioethern, Aminen, Phosphinen, bevorzugt Tetrahydrofuran (THF), 2-Methyltetrahydrofuran, Dibutylether, Diethylether, tert-Butylmethylether, Dimethoxyethan, Dioxane, bevorzugt 1,4-Dioxan, Triethylamin, Ethyldi*iso*propylamin, Dimethylsulfid, Dibutylsulfid; cyclische Amide, bevorzugt N-Methyl-2-pyrrolidon (NMP), *N*-Ethyl-2-pyrrolidon (NEP), *N*-Butyl-2-pyrrolidon (NBP); cyclische, unverzweigte oder verzweigte Haloalkane und Haloalkene, bevorzugt Dichlormethan, 1,2-Dichlorethan, CCl₄; Harnstoffe, bevorzugt *N*,*N*'-Dimethylpropylenharnstoff (DMPU); aromatische, heteroaromatische oder aliphatische Kohlenwasserstoffe, bevorzugt Benzol, Toluol, Xylol, Pyridin, Pentan, Cyclohexan, Hexan, Heptan; Hexamethylphosphorsäuretriamid (HMPA), CS₂ ausgewählt ist.

Besonders bevorzugt liegt die metallorganische Verbindung des iso-TEMPs der allgemeinen Formel Me_{A}^{m1}[R_{iso-TEMP}]ₙ₁Y¹ₙ₂·zMe_{B}^{m2}Y²ₙ₃ in einem Lösungsmittel vor, welches aus der Gruppe bestehend aus Tetrahydrofuran (THF), 2-Methyltetrahydrofuran, Dibutylether, Diethylether, tert-Butylmethylether, Dimethoxyethan, 1,4-Dioxan, Triethylamin, Ethyldi*iso*propylamin, Dimethylsulfid, Dibutylsulfid, *N*-Methyl-2-pyrrolidon (NMP), *N*-Ethyl-2-pyrrolidon (NEP), *N*-Butyl-2-pyrrolidon (NBP), Dichlormethan, 1,2-Dichlorethan, CCl₄, *N*,*N*'-Dimethylpropylenharnstoff (DMPU), Benzol, Toluol, Xylol, Pyridin, Pentan, Cyclohexan, Hexan, Heptan; Hexamethylphosphorsäuretriamid (HMPA), CS₂ ausgewählt ist und ganz besonders bevorzugt THF oder Diethylether, noch bevorzugter THF ist.

Wie in den nachfolgenden Beispielen gezeigt, eignet sich diese Verbindung zur Verwendung in einer Reaktion mit einem Elektrophil oder zur Deprotonierung von Substraten, die stabilisierte oder unstabilisierte Carbanionen bilden können. Dabei zeigen die aus dem Grignardverbindung hergestellten Verbindungen ähnliche Aktivitäten wie die mit den entsprechenden Lithium-Magnesium-Addukten hergestellten metallorganischen Verbindungen.

Die nachfolgenden Beispiele sollen die Erfindung illustrieren, ohne diese zu beschränken.

### Beispiele

### 1. Erhalt des Destillats aus einem Rohproduktstrom der TAA-Synthese

Zwei in Reihe geschaltete zylindrische Reaktoren wurden mit Lewatit K2621 (Polystyrol-Katalysator mit -SO₃⁻ als funktionelle Gruppe; von Lanxess) so gefüllt, dass dieses Katalysatormaterial durch Siebe oben und unten begrenzt, angeordnet war. Das Schüttvolumen des Katalysators betrug je 600 mL im wasserfeuchten Zustand. Der Reaktor wurde kontinuierlich mit 630 g/h Aceton und 25 g/h Ammoniak beschickt. Dabei wurde eine Temperatur von 75 °C und ein Druck von 14 bar eingestellt. Der Zulauf betrug insgesamt 961.83 g (16.58 mol) Aceton. Die LHSV (liquid hourly space velocity, also das Volumen an Reaktanden die pro Stunde bezogen auf das Reaktorvolumen zugegeben wurden), betrug im Falle des Ammoniaks 0.03 bis 0.06 h⁻¹, und im Falle des Acetons 0.66 bis 1.33 h⁻¹.

1 kg des Reaktoraustrages wurde mit 3 bis 5 Gew.-% Wasser, bezogen auf die ursprünglich eingesetzte Menge an Aceton, versetzt und anschließend destillativ an einer Rektifikationskolonne aufgearbeitet. Dabei wurde so lange destilliert, dass eine Abtrennung sämtlicher Leichtsieder (Aceton, Mesityloxid, Diacetonalkohol, Diacetonamin) gelang, TMDHPyridin und TAA und weitere entstandene Hochsieder jedoch als Abfall- bzw. Wertprodukt über den Sumpf abgetrennt wurden. Zwischensieder, wie Acetonin und Phoron, wurden zu einem möglichst hohen Anteil in leichtsiedende Komponenten hydrolisiert und waren dadurch abtrennbar.

Danach wurde der Anteil an den verschiedenen Komponenten im Sumpf und im Destillat per GC [Säule: HP-50 (30m), Temperaturprogramm: 5 °C/min 60 - 130°C, 15 °C/min - 270°C, 10 min] bestimmt. Der relative Anteil der jeweiligen Komponenten im Reaktoraustrag, Destillat und Sumpf wurde bestimmt und ist in der folgenden Tabelle 1 aufgeführt. In der folgenden Tabelle 1 sind (mit Ausnahme des Wassers) die Komponenten im Reaktionsaustrag von links nach rechts gemäß ihrer Siedepunkte aufgeführt ("n.i." = nicht identifiziert).

Die ersten drei Zeilen geben die Flächenprozente (inklusive Wasser) der jeweiligen Komponente im GC-Diagramm wieder.

Die letzte Zeile ergibt sich wie folgt:
Basierend auf der Gesamtmasse des Sumpfes (200 g) wurden die Massen der jeweiligen Komponente im Sumpf aus dem prozentualen Anteil der jeweiligen Verbindung im GC ermittelt. Daraus lässt sich die Stoffmenge der jeweiligen Komponente im Sumpf und somit auch die Stoffmenge an Acetonäquivalenten berechnen, denen die jeweilige Komponente entspricht.

Dabei entspricht Aceton einem Acetonäquivalent.

Dabei entsprechen die folgenden Verbindungen zwei Acetonäquivalenten: Mesityloxid (und Isomer), Diacetonalkohol, Diacetonamin.

Dabei entsprechen die folgenden Verbindungen drei Acetonäquivalenten: Acetonin, Phoron, Isophoron.

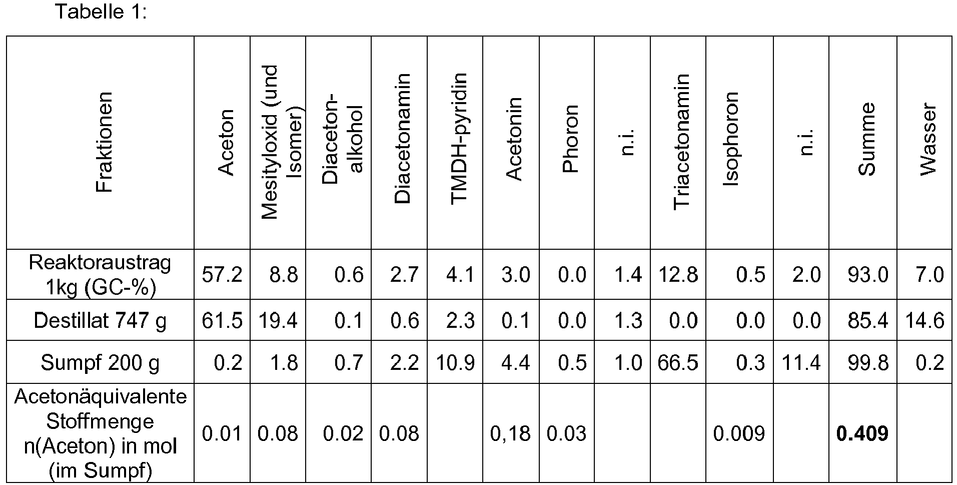

### 2. Destillation des Sumpfstroms unter Erhalt des Kopfstroms 1

Der unter Punkt 1 erhaltene Sumpfstrom wurde in einer weiteren Rektifikationskolonne destilliert, um TMDH-Pyridin abzutrennen (100-110 °C Sumpf, 70-100 °C Kopf, 160-50 mbar Kopf). Es wurden 10 Fraktionen genommen
Der erhaltene Kopfstrom (im Folgenden "Kopfstrom 1") wurde gaschromatographisch analysiert. Die GC-Methode war die unter Punkt 1 angegebene. Der Kopfstrom enthielt folgende Komponenten ("n.i."= nicht identifiziert).

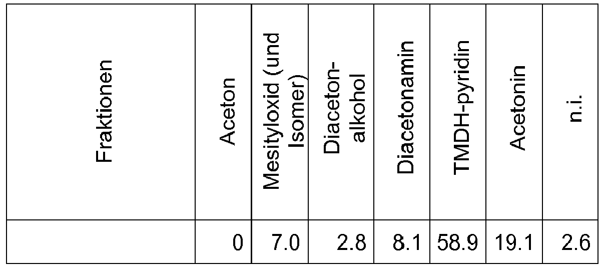

### 3. Destillation des Kopfstromes 1 unter Erhalt des Kopfstroms 2

Aus dem in Punkt 2 erhaltene Kopfstrom 1 wurde erneut TMDH-Pyridin destilliert (Temperatur des Sumpfs: 100 bis 110 °C, Temperatur am Kopf, 90 bis 100 °C, Druck 120 bis 180 mbar). Es wurden alle Fraktionen, die mehr als 80 Gew.-% TMDH-Pyridin enthielten, vereinigt und die Zusammensetzung dieser Destillate (im Folgenden "Kopfstrom 2") gaschromatographisch wie unter Punkt 2 beschrieben analysiert:

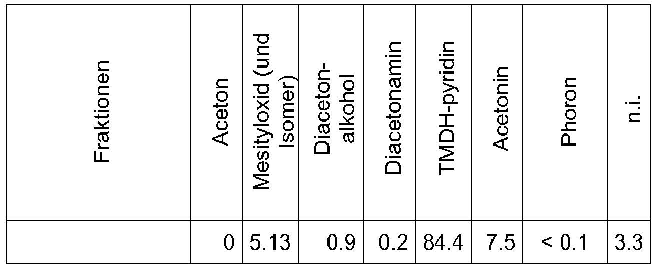

Das entsprechende Chromatogramm des Kopfstroms 2 ist in Figur 1 gezeigt.

### 4. Hydrierung

### 4.1 Hydrierung des Kopfstroms 1

Der unter Punkt 2 erhaltene Kopfstrom 1 wurde dann direkt in einem Batch-Autoklaven für 35 bis 45 Stunden bei 20 bis 25 bar H₂ und 140 °C hydriert. Als Katalysator diente Palladium auf Kohle (10 Gew.%). Ein Lösungsmittel wurde nicht hinzugefügt.

### 4.2 Hydrierung des Kopfstroms 2

20 kg des unter Punkt 3 erhaltene Kopfstrom 2 wurde in einem Batch-Autoklaven für 36 bis 48 Stunden bei 20 bis 25 bar H₂ und 60 °C bis 140 °C hydriert. Als Katalysator diente Palladium auf Kohle (10 Gew.%). Als Lösungsmittel wurden 29 kg Methanol eingesetzt.

### 5. Destillation des iso-TEMPs

5.1 Das in Punkt 4.1 erhaltene Rohprodukt wurde fraktioniert in einer Kolonne mit strukturierter Packung destilliert (Sumpf: 100 bis 120 °C, Druck: nicht detektierbar bis 100 mbar, Kopftemperatur 60 bis 85 °C). Es wurden insgesamt 10 Fraktionen genommen, und der Inhalt jeder Fraktion wurde gaschromatographisch vermessen.

Das entsprechende Chromatogramm ist in Figur 2 gezeigt.

5.2 Das in Punkt 4.2 erhaltene Rohprodukt wurde wiederum fraktioniert destilliert. Es wurden insgesamt 13 Fraktionen genommen, und der Inhalt jeder Fraktion wurde gaschromatographisch vermessen. Das entsprechende Chromatogramm ist in Figur 3 gezeigt.

Aus dem Vergleich des in Figur 1 gezeigten Gaschromatogramms mit den in Figur 2 und 3 gezeigten Gaschromatogrammen ergibt sich:
- Aus Figur 1 ist ersichtlich, dass sich die Peaks von Mesityloxid und TMDH-Pyridin in fünf Fraktionen überschneiden. Die Abtrennung des einen Stoffs vom anderen ist nur schwer möglich.
- Demgegenüber überlappen die Peaks von 4-Methyl-pentan-2-on und *iso*-TEMP nur in zwei Fraktionen, wie sowohl in Figur 2 als auch Figur 3 ersichtlich.
- Es zeigte sich somit überraschend, dass eine saubere Fraktion *iso*-TEMP erhalten werden konnte, wenn nach dem erfindungsgemäßen Verfahren vorgegangen wird.
- Demgegenüber ist eine Abtrennung von TMDH-Pyridin von Mesityloxid nur schwer möglich.
- Zusätzlich ermöglicht das erfindungsgemäße Verfahren außerdem, 4-Methyl-pentan-2-on viel reiner, als dies im Falle des Mesityloxids möglich wäre, als Rohprodukt zu erhalten.

### 6. Einsatz von iso-TEMP als Elektrophil

Die Eigenschaft von *iso*-TEMP, als Elektrophil zu fungieren, wurde anhand eines Testsystems untersucht. Zu diesem Zweck wurde 2,2-Difluoro-1,3-benzodioxole (DFBD, CAS-No.: 1583-59-1) mit der aus dem jeweiligen Amin erhaltenen Grignard-Verbindung umgesetzt. Letztere wurde mit verschiedenen Aminen R*NH entsprechend der Reaktion

*iso*-PrMgCl•LiCl + R*NH → R*NMgCl•LiCl + *iso*-PrH oder

MeMgCI + R*NH → R*NMgCl + MeH

hergestellt, wobei R* für den entsprechenden aliphatischen oder aromatischen Rest des jeweiligen Amins steht. Durch Reaktion der so erhaltenen Grignardverbindung mit DFBD und Abfangen der Zwischenstufe mit lod konnte das entsprechende lodderivat des DFBD [abgekürzt als DFBD(I)] erhalten werden. Die Bestimmung des Gehalts des lodderivats gibt direkt Auskunft über die Effizienz des jeweiligen Amins in der Testreaktion.

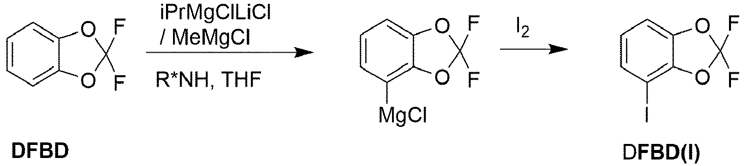

Es wurden als Amine die in der folgenden Tabelle gezeigten Amine getestet, also TEMP, *iso*-TEMP, Diphenylamin, Di*iso*propylamin, *tert*-Butylamin, Dibutylamin, Diethylamin, Pyrrol, Piperidin, Diallylamin.

In jedem Versuch wurden 0.06 mol der jeweiligen Grignard-Verbindung (*i*PrMgCl•LiCl bzw. MeMgCl) in 50 mL THF unter Stickstoffatmosphäre gelöst und anschließend 0.06 mol des wasserfreien Amins hinzugetropft. Die resultierende Mischung wurde über Nacht bei Raumtemperatur gerührt.

In einem zweiten Kolben wurden 0.05 Mol (7.91 g) DFBD in 25 mL THF gelöst und die am Vortag angesetzte Grignard/Amin-Mischung langsam über 30 Minuten hinzugetropft. Es wurde mit 5 mL THF nachgewaschen. Anschließend wurde die Mischung bei Raumtemperatur gerührt. Nach vier sowie 24 Stunden wurde eine Probe (3 mL) entnommen, diese mit 2 mL einer 1 M-Iodlösung bei 0 °C versetzt und anschließend für 30 Minuten gerührt. Anschließend wurde eine gesättigte, wässrige Ammoniumchlorid-Lösung (2 mL) hinzugegeben und 2 mL Wasser hinzugefügt. Es wurde auf Raumtemperatur erwärmt, ehe 2.5 mL Toluol zugegeben und die Phasen im Scheidetrichter getrennt wurden. Die organische Phase wurde anschließend mittels Gaschromatographie hinsichtlich des Umsatzes an DFBD(I) nach 4 Stunden analysiert. Der erfinderische Versuch ist fett gedruckt, die anderen Versuche entsprechen Vergleichen.

| **Base** | **Grignard-Reagenz** | **Umsatz** |
|---|---|---|
| TEMP | *i*PrMgCl•LiCl | 66 % |
| | MeMgCl | 41 % |
| ***iso*-TEMP** | ***i*PrMgCl•Cl** | **37 %** |
| | **MeMgCl** | **35 %** |
| Diphenylamin | *i*PrMgCl•LiCl | 0 % |
| | MeMgCl | 0 % |
| Diisopropylamin | *i*PrMgCl•LiCl | 39 % |
| | MeMgCl | 41 % |
| *tert*-Butylamin | *i*PrMgCl•LiCl | 0 % |
| | MeMgCl | 0 % |
| Dibutylamin | *i*PrMgCl•LiCl | 0 % |
| | MeMgCl | 0 % |
| Diethylamin | *i*PrMgCl•LiCl | 0 % |
| | MeMgCl | 0 % |
| Pyrrol | *i*PrMgCl•LiCl | 0 % |
| | MeMgCl | 0 % |
| Piperidin | *i*PrMgCl•LiCl | 0 % |
| | MeMgCl | 0 % |
| Diallylamin | *i*PrMgCl•LiCl | 0 % |
| | MeMgCl | 0 % |

Aus den in der Tabelle dargestellten Versuchsergebnissen geht hervor, dass mit *iso*-TEMP eine gute Ausbeute zum Produkt DFBD(I) erhalten werden konnte. DFBD(I) fungiert damit überraschend gut als Elektrophil.

## Patentansprüche

1. Verfahren zur Herstellung von Triacetonamin, 2,2,4,6-Tetramethylpiperidin und/oder einer metallorganischen Verbindung des 2,2,4,6-Tetramethylpiperidins, umfassend die folgenden Schritte
(a) Umsetzen von Ammoniak und Aceton in Gegenwart eines Katalysators **K₁** zu einem Rohprodukt **RP₁** umfassend Triacetonamin, 2,2,4,6-Tetramethyl-2,3-dihydropyridin und Mesityloxid,
(b) mindestens teilweise Abtrennung eines Rohprodukts **RP₁₁** umfassend 2,2,4,6-Tetramethyl-2,3-dihydropyridin und Mesityloxid aus **RP₁,** wobei **RP₁₁** eine gegenüber **RP₁** verringerte Menge an Triacetonamin aufweist,
**dadurch gekennzeichnet, dass**
(c) **RP₁₁** mindestens teilweise zu einem Rohprodukt **RP₁₂** umfassend 2,2,4,6-Tetramethylpiperidin und 4-Methyl-pentan-2-on umgesetzt wird,
(d) das in **RP₁₂** enthaltenen 2,2,4,6-Tetramethylpiperidin durch Destillation mindestens teilweise vom in **RP₁₂** enthaltenen 4-Methyl-pentan-2-on abgetrennt wird.

2. Verfahren nach Anspruch 1, wobei die mindestens teilweise Abtrennung von **RP₁₁** aus **RP₁** destillativ erfolgt.

3. Verfahren nach Anspruch 2, wobei in Schritt (b)
(i) aus dem Rohprodukt **RP₁** zunächst teilweise Mesityloxid destillativ abgetrennt werden und so ein Rohprodukt **RP₁₁₁** erhalten wird, so dass der Gesamtgehalt an Mesityloxid in **RP₁₁₁** gegenüber dem Gesamtgehalt an Mesityloxid in **RP₁** verringert ist,
(ii) dann aus **RP₁₁₁** destillativ 2,2,4,6-Tetramethyl-2,3-dihydropyridin zusammen mit Mesityloxid als Destillat **RP₁₁₂** abgetrennt wird und
(iiiA) **RP₁₁₂** als **RP₁₁** dem Schritt (c) zugeführt wird, oder
(iiiB) aus **RP₁₁₂** teilweise 2,2,4,6-Tetramethyl-2,3-dihydropyridin, welches Mesityloxid umfasst, als Destillat **RP₁₁₃** destilliert wird, und dann **RP₁₁₃** als **RP₁₁** dem Schritt (c) zugeführt wird.

4. Verfahren nach Anspruch 2 oder 3, wobei der bei der Destillation in Schritt (b) eingesetzte Temperaturbereich unterhalb der Siedetemperatur von Triacetonamin liegt.

5. Verfahren nach einem der Ansprüche 1 bis 4, wobei der Schritt (a) bei einer Temperatur von 20 °C bis 180 °C durchgeführt wird.

6. Verfahren nach einem der Ansprüche 1 bis 5, wobei das Molverhältnis von in Schritt (a) eingesetztem Aceton zu in Schritt (a) eingesetztem Ammoniak 3 : 1 bis 20 : 1 beträgt.

7. Verfahren nach einem der Ansprüche 1 bis 6, wobei **K₁** ein heterogener Katalysator ist.

8. Verfahren nach einem der Ansprüche 1 bis 7, wobei **K₂** ein Träger- oder Vollkatalysator ist.

9. Verfahren nach einem der Ansprüche 1 bis 8, wobei Schritt (c) bei einer Temperatur von 20 °C bis 300 °C und einem Druck von 1 bis 300 bar durchgeführt wird.

10. Verfahren nach einem der Ansprüche 1 bis 9 zur Herstellung einer metallorganischen Verbindung des 2,2,4,6-Tetramethylpiperidins,
wobei das in Schritt (d) abgetrennte 2,2,4,6-Tetramethylpiperidin in einem Schritt (e) mit einem Reagenz der Formel Me_{A}^{m1}Rₙ₁Y¹ₙ₂·zMe_{B}^{m2}Y²ₙ₃ in einem Lösungsmittel umgesetzt wird,
wobei m1, n1, m2, n3 unabhängig voneinander jeweils eine ganze Zahl > 0 sind,
und wobei n2, z unabhängig voneinander jeweils eine ganze Zahl ≥ 0 sind,
wobei m1 die Wertigkeit des Metalls Me_{A} angibt und wobei m2 die Wertigkeit des Metalls Me_{B} angibt, wobei gilt, dass m1 + z·m2 = n1 + n2 + z·n3,
wobei R ein Kohlenwasserstoffrest aus gewählt aus Alkylgruppe, Alkenylgruppe, Arylgruppe, Alkinylgruppe ist,
wobei Me_{A}, Me_{B} unabhängig voneinander jeweils ein Metall ausgewählt aus der Gruppe bestehend aus Li, Mg, Zn, Zr, Ti, Cu, Au, Ag, Hg, Cd ist,
und wobei Y¹ und Y² unabhängig voneinander aus der Gruppe bestehend aus F, Cl, Br, I, CN, SCN, NCO, ClO₃, ClO₄, BrO₃, BrO₄, IO₃, IO₄₁ NO₃, BF₄, PF₆, Wasserstoff, ein Carboxylat der allgemeinen Formel R^{x}CO₂, ein Alkoholat der allgemeinen Formel OR^{x}; ein Thiolat der allgemeinen Formel SR^{x}, R^{x}P(O)O₂, or SCOR^{x}, SCSR^{x}, O₂SR^{x}, O₃SR^{x}, NO₂ ausgewählt sind,
wobei R^{x} ausgewählt ist aus der Gruppe bestehend aus unsubstituierter oder substituierter Arylest oder Heteroarylrest mit einem oder mehreren Heteroatomen, verzweigter oder unverzweigter Alkylrest, Cycloalkylrest, Alkenylrest, Alkinylrest, Wasserstoff oder als Addukt mit einem Lösungsmittel,
wodurch eine metallorganische Verbindung des 2,2,4,6-Tetramethylpiperidins der allgemeinen Formel Me_{A}^{m1}[R_{iso-TEMP}]ₙ₁Y¹ₙ₂·zMe_{B}^{m2}Y²ₙ₃ mit R_{iso-TEMP} = 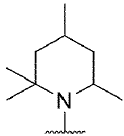 erhalten wird.

11. Verfahren nach Anspruch 10, wobei Schritt (e) bei einer Temperatur im Bereich von - 50 °C bis 25 °C durchgeführt wird.

12. Verfahren nach Anspruch 10 oder 11, wobei das Lösungsmittel ausgewählt ist aus der Gruppe bestehend aus Tetrahydrofuran, 2-Methyltetrahydrofuran, Dibutylether, Diethylether, *tert*-Butylmethylether, Dimethoxyethan, 1,4-Dioxan, Triethylamin, Ethyldi*iso*propylamin, Dimethylsulfid, Dibutylsulfid, *N*-Methyl-2-pyrrolidon, *N*-Ethyl-2-pyrrolidon, *N*-Butyl-2-pyrrolidon, Dichlormethan, 1,2-Dichlorethan, CCl₄, *N*,*N*'-Dimethylpropylenharnstoff, Benzol, Toluol, Xylol, Pyridin, Pentan, Cyclohexan, Hexan, Heptan, Hexamethylphosphorsäuretriamid, CS₂.

13. Verbindung der allgemeinen Formel Me_{A}^{m1}[R_{iso-TEMP}]ₙ₁Y¹ₙ₂·zMe_{B}^{m2}Y²ₙ₃
mit R_{iso-TEMP} = 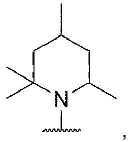 wobei m1, n1, m2, n3 unabhängig voneinander jeweils eine ganze Zahl > 0 sind, und wobei n2, z unabhängig voneinander jeweils eine ganze Zahl ≥ 0 sind,
wobei m1 die Wertigkeit des Metalls Me_{A} angibt und wobei m2 die Wertigkeit des Metalls Me_{B} angibt, wobei gilt, dass m1 + z·m2 = n1 + n2 + z·n3,
wobei Me_{A}, Me_{B} unabhängig voneinander jeweils ein Metall ausgewählt aus der Gruppe bestehend aus Li, Mg, Zn, Zr, Ti, Cu, Au, Ag, Hg, Cd, sind,
und wobei Y¹ und Y² unabhängig voneinander aus der Gruppe bestehend aus F, Cl, Br, I, CN, SCN, NCO, ClO₃, ClO₄, BrO₃, BrO₄, IO₃, IO₄₁ NO₃, BF₄, PF₆, Wasserstoff, ein Carboxylat der allgemeinen Formel R^{x}CO₂, ein Alkoholat der allgemeinen Formel OR^{x}; ein Thiolat der allgemeinen Formel SR^{x}, R^{x}P(O)O₂, or SCOR^{x}, SCSR^{x}, O₂SR^{x}, O₃SR^{x}, NO₂ ausgewählt sind,
wobei R^{x} ausgewählt ist aus der Gruppe bestehend aus unsubstituierter oder substituierter Arylest oder Heteroarylrest mit einem oder mehreren Heteroatomen, verzweigter oder unverzweigter Alkylrest, Cycloalkylrest, Alkenylrest, Alkinylrest, Wasserstoff oder als Addukt mit einem Lösungsmittel.

14. Verbindung nach Anspruch 13, wobei das Lösungsmittel ausgewählt ist aus der Gruppe bestehend aus Tetrahydrofuran, 2-Methyltetrahydrofuran, Dibutylether, Diethylether, *tert*-Butylmethylether, Dimethoxyethan, 1,4-Dioxan, Triethylamin, Ethyldi*iso*propylamin, Dimethylsulfid, Dibutylsulfid, *N*-Methyl-2-pyrrolidon, *N*-Ethyl-2-pyrrolidon, *N*-Butyl-2-pyrrolidon, Dichlormethan, 1,2-Dichlorethan, CCl₄, *N*,*N*'-Dimethylpropylenharnstoff, Benzol, Toluol, Xylol, Pyridin, Pentan, Cyclohexan, Hexan, Heptan, Hexamethylphosphorsäuretriamid, CS₂.
